# FASCICULE DE BREVET EUROPEEN

(11) **EP 3 280 706 B1**
(45) Date de publication et mention de la délivrance du brevet: **22.05.2019**
(21) Numéro de dépôt: 16722280.1
(22) Date de dépôt: 05.04.2016
(51) Int. Cl.: C07D 317/38, C08G 61/08, C08G 61/12, C08G 71/04

(54) **POLYMÈRES HYDROCARBONÉS COMPRENANT DEUX GROUPEMENTS TERMINAUX EXO-VINYLÈNE CYCLOCARBONATE**
KOHLENWASSERSTOFFPOLYMERE MIT ZWEI CYCLISCHEN EXO-VINYLEN-CARBONATENDGRUPPEN
HYDROCARBON POLYMERS COMPRISING TWO EXO-VINYLENE CYCLIC CARBONATE TERMINAL GROUPS

(30) Priorité: 10.04.2015 FR 1553103
(43) Date de publication de la demande: 14.02.2018
(73) Titulaire: Bostik SA, 92700 Colombes (FR)
(72) Inventeur: MICHAUD, Guillaume, 60200 Compiegne (FR); SIMON, Frédéric, 60400 Pont l'Eveque (FR); FOUQUAY, Stéphane, 76130 Mont Saint Aignan (FR)
(74) Mandataire: Chahine, Audrey Claire
(86) Numéro de dépôt international: PCT/FR2016/050774
(87) Numéro de publication internationale: WO 2016/162627

(56) Documents cités:
- EP-A1- 2 851 379
- EP-A1- 2 851 403
- WO-A1-2013/144299
- WO-A1-2014/091173
- DE-B- 1 098 953
- US-A1- 2013 331 532

## Description

La présente invention a pour objet des polymères hydrocarbonés comprenant deux groupements terminaux exo-vinylène cyclocarbonate (aussi désignés polymères hydrocarbonés à terminaisons exo-vinylène cyclocarbonate dans la présente demande).

L'invention concerne aussi l'utilisation de ces polymères hydrocarbonés pour la préparation de polyuréthanes, sans isocyanate, par réaction avec au moins un composé comprenant au moins un groupe amine. Ces polyuréthanes, une fois formulés, sont destinés à être utilisés dans des compositions de revêtements, de mastics ou d'adhésifs.

La synthèse de polyuréthanes se fait traditionnellement par réaction entre un diol et un diisocyanate. Les diisocyanates sont des composés toxiques en tant que tels, et sont généralement obtenus à partir de phosgène, lui-même très toxique par inhalation ou par contact. Le procédé de fabrication utilisé dans l'industrie met généralement en oeuvre la réaction d'une amine avec un excès de phosgène pour former un isocyanate.

La recherche d'alternatives à la synthèse de polyuréthanes sans utiliser d'isocyanate (ou NIPU pour « Non Isocyanate PolyUrethane » en anglais, c'est-à-dire « polyuréthane sans isocyanate »), représente donc un enjeu majeur.

Cette recherche a fait l'objet de nombreuses études. Les pistes les plus étudiées concernent l'utilisation de polymères susceptibles de réagir avec des amines ou oligomères d'amines pour former des polyuréthanes.

La demande de brevet WO 2014/091173 au nom de Bostik et du CNRS, décrit des polymères hydrocarbonés comprenant deux groupements terminaux à terminaison (2-oxo-1,3-dioxolan-4-yl) susceptibles d'être obtenus par polymérisation par ouverture de cycle par métathèse à partir d'au moins une cyclooléfine cyclique, au moins un agent de transfert de chaine insaturé non cyclique comprenant un groupement terminal (2-oxo-1,3-dioxolan-4-yl), et au moins un catalyseur de métathèse.

Ces polymères peuvent ensuite réagir avec une (poly)amine pour former des polyuréthanes sans isocyanate qui peuvent être avantageusement utilisés pour formuler des compositions de revêtements, de mastics ou d'adhésifs. Toutefois, cette réaction est relativement longue et reste à améliorer.

L'exemple 4 de la demande de brevet WO 2014/091173 divulgue notamment la réaction d'une polyoléfine insaturée comprenant deux groupements terminaux (2-oxo-1,3-dioxolan-4-yl)méthyl oxycarbonyle avec une diamine primaire de type polyéther diamine, utilisés en proportions stoechiométriques, pour former un polyuréthane pouvant être formulé sous la forme d'une composition adhésive bi-composant. La durée de cette réaction est de 12h à 80°C.

Afin de remédier aux inconvénients divulgués dans la demande de brevet WO 2014/091173, il est proposé d'utiliser de nouveaux intermédiaires permettant la synthèse de polyuréthanes sans utiliser d'isocyanate, destinés notamment à la fabrication de compositions de revêtements, de mastics ou d'adhésifs.

Ainsi, la présente invention concerne un polymère hydrocarboné comprenant deux groupements terminaux exo-vinylène cyclocarbonate, ledit polymère hydrocarboné étant de formule (1) suivante : 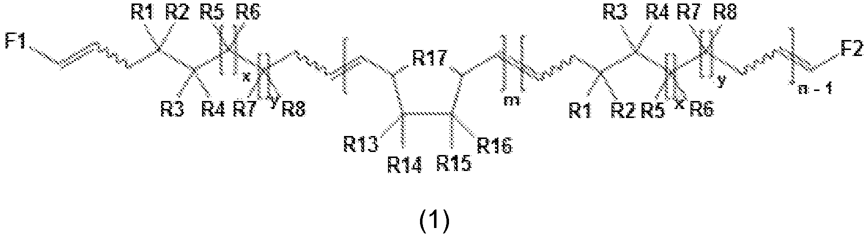 dans laquelle :
- chaque liaison notée 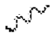 est une liaison simple carbone-carbone orientée géométriquement d'un côté ou de l'autre par rapport à la double liaison (cis ou trans) à laquelle elle est reliée ;
- les groupes R1, R2, R3, R4, R5, R6, R7 et R8, identiques ou différents, sont choisis parmi un atome d'hydrogène, un atome d'halogène, un groupe alkyle, un groupe hétéroalkyle, un groupe alcoxycarbonyle ou un groupe hétéroalcoxycarbonyle,
- au moins un, et de préférence un, des groupes R1 à R8 pouvant faire partie d'un même cycle hydrocarboné ou hétérocycle, saturé ou insaturé, avec au moins un autre, et de préférence avec un autre, des groupes R1 à R8, selon les règles de valence de la chimie organique ;
- au moins une des paires (R1,R2), (R3,R4), (R5,R6) et (R7,R8) pouvant être un groupe oxo ;
- x et y, identiques ou différents, sont des nombres entiers compris dans une fourchette allant de 0 à 5, de préférence allant de 0 à 2, de façon encore plus préférée x est égal à 1 et y est égal à 1, la somme x + y étant de préférence comprise dans une fourchette allant de 0 à 4 et de façon encore plus préférée allant de 0 à 2 ;
- les groupes R13, R14, R15 et R16 identiques ou différents, sont choisis parmi un atome d'hydrogène, un atome d'halogène, un groupe alkyle, un groupe hétéroalkyle, un groupe alcoxycarbonyle ou un groupe hétéroalcoxycarbonyle,
- au moins un des groupes R13 à R16 pouvant faire partie d'un même cycle hydrocarboné ou hétérocycle, saturé ou insaturé, avec au moins un autre des groupes R13 à R16, selon les règles de valence de la chimie organique ;
- le groupe R17 est CH₂, O, S, C(=O) ou NR₀, R₀ étant un groupe alkyle, de préférence linéaire, comportant de 1 à 22, de préférence de 1 à 14, atomes de carbone ; et
- n est un nombre entier supérieur ou égal à 2 et m est un nombre entier supérieur ou égal à 0, le rapport molaire m/n étant compris dans une fourchette allant de 0/100 à 90/10, de préférence allant de 25/100 à 75/50, et étant plus préférentiellement égal à 50/50; n et m étant en outre tels que la masse molaire moyenne en nombre Mn du polymère hydrocarboné de formule (1) est comprise dans une fourchette allant de 400 à 50 000 g/mol, de préférence allant de 600 à 20 000 g/mol, et la polymolécularité (PDI) du polymère hydrocarboné de formule (1) est comprise dans une fourchette allant de 1,0 à 3,0, de préférence allant de 1,0 à 2,0, de façon encore plus préférée allant de 1,45 à 1,85 ;
- F1 est représenté par la formule suivante : 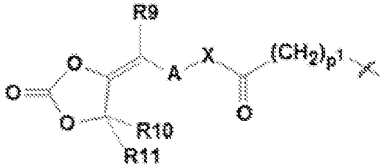
- et F2 est représenté par la formule suivante : 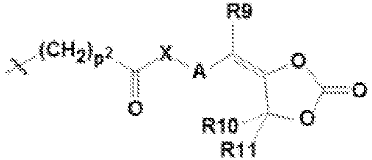 dans lesquelles :
   p1 et p2, identiques ou différents, représentent chacun un nombre entier égal à 0, 1, 2 ou 3, avec de préférence p1 = 0 ou p2 = 0, et plus préférentiellement p1 = p2 = 0 ;
   X est un atome d'oxygène ou un groupe azoté NR12 où R12 est un groupe alkyle en C1-C6 ;
   A est un groupe alkylène en C1-C6 ;
   R9 est un atome d'hydrogène, un groupe alkyle en C1-C6, un groupe alkyle en C1-C6 oxyalkyléné par un ou plusieurs groupes oxyalkylène en C1-C6, un groupe cycloalkyle en C5-C6, un groupe phényle ou un groupe alkylphényle avec une chaîne alkyle en C1-C4 ;
   R10 et R11, identiques ou différents, représentent chacun un atome d'hydrogène, un groupe alkyle en C1-C6, un groupe alkyle en C1-C6 oxyalkyléné par un ou plusieurs groupes oxyalkylène en C1-C6, un groupe cycloalkyle en C5-C6, un groupe phényle ou un groupe alkylphényle avec une chaîne alkyle en C1-C4.

De préférence, le groupe alkyle en C1-C6 oxyalkyléné par un ou plusieurs groupes oxyalkylène en C1-C6 tel qu'envisagé dans la définition de R9, R10 et R11 précédemment est un groupe alkyle en C1-C4 oxyalkyléné par un ou plusieurs groupes oxyalkylène en C1-C4 (tel que oxyméthylène, oxyéthylène, oxypropylène ou oxybutylène).

Bien évidemment, toutes les formules sont données ici en accord avec les règles de valence de la chimie organique.

La chaîne principale du polymère de formule (1) comporte donc un ou deux types d'unités de répétition, un premier type d'unité de répétition répété n fois et un second type d'unité de répétition, optionnel, répété m fois.

Ainsi qu'il apparaît ci-dessus, les groupements terminaux F1 et F2 sont généralement symétriques par rapport à la chaîne principale, c'est-à-dire qu'ils se correspondent sensiblement, à l'exception des indices p1 et p2.

Dans la présente demande, en l'absence d'indication contraire :
- Par « groupement terminal », on entend un groupement situé en bout de chaîne (ou extrémité de la chaine principale) du polymère. Le polymère selon l'invention comprend une chaîne principale, c'est-à-dire une chaîne plus longue, dont les deux extrémités sont les groupements terminaux du polymère selon l'invention.
- Par « groupement exo-vinylène cyclocarbonate », on entend un groupe F1 ou F2 tel que décrit précédemment.
- Par « groupe alkyle », on entend un composé hydrocarboné linéaire ou ramifié, cyclique (incluant polycyclique) ou acyclique, et comprenant sauf indication contraire généralement de 1 à 22 atomes de carbone. Un tel groupe alkyle comprend le plus souvent de 1 à 14, de préférence de 1 à 8, atomes de carbone.
- Par « groupe hétéroalkyle », on entend selon l'invention un groupe alkyle dans lequel au moins un des atomes de carbone est substitué par un hétéroatome choisi dans le groupe formé par O et S.
- Par « groupe alcoxycarbonyle », on entend un groupe (monovalent) alkyle, linéaire ou ramifié, saturé ou partiellement insaturé, comprenant de 1 à 22, de préférence de 1 à 14, atomes de carbone, ainsi qu'un groupe divalent - COO-.
- Par « groupe hétéroalcoxycarbonyle », on entend selon l'invention un groupe alcoxycarbonyle dans lequel au moins un des atomes de carbone est substitué par un hétéroatome choisi dans le groupe formé par O et S.
- Par « atome d'halogène », on entend un groupe iodo, chloro, bromo ou fluoro, de préférence chloro.
- Par « cycle hydrocarboné », on entend un composé hydrocarboné cyclique (incluant polycyclique), saturé ou insaturé, pouvant comporter de 3 à 22 atomes de carbone et éventuellement au moins un groupe C=O.
- Par « hétérocycle », on entend un cycle hydrocarboné pouvant comprendre un autre atome que le carbone dans la chaîne du cycle, tel que par exemple l'oxygène, le soufre ou le groupe NR₀ tel que défini précédemment, à savoir un groupe alkyle, de préférence linéaire, comportant de 1 à 22, de préférence de 1 à 14, atomes de carbone.
- Par « au moins un des groupes R1 à R8 pouvant faire partie d'un même cycle hydrocarboné ou hétérocycle, saturé ou insaturé, avec au moins un autre des groupes R1 à R8, selon les règles de valence de la chimie organique », on entend selon l'invention que ces groupes, qu'ils soient portés ou non par le même carbone, sont liés ensemble par une chaîne hydrocarbonée (pouvant inclure les atomes de carbone de la chaine principale du polymère de formule (1)) comportant éventuellement au moins un groupe un groupe C=O et/ou au moins un hétéroatome tel que S ou O, de manière à former un cycle hydrocarboné ou un hétérocycle, tels que définis ci-dessus. Dans certains cas, ces groupes peuvent désigner des liaisons chimiques. Par exemple, lorsque l'un des groupes R3 ou R4 avec l'un des groupes R5 ou R6 forment ensemble avec les atomes de carbone de la chaine principale du polymère de formule (1) les supportant, un cycle époxyde, ceux-ci désignent une liaison reliant l'atome de carbone les supportant à l'atome d'oxygène du groupe époxy. Ces définitions sont également applicables aux groupes R13 à R16.
- Par « paire (R1,R2) pouvant être un groupe oxo », on entend selon l'invention que la paire (R1,R2) est telle que 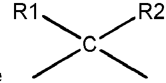 est 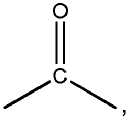 où C est l'atome de carbone qui supporte les deux groupes formant la paire (R1 ,R2). Cela est également applicable aux paires (R3,R4), (R5,R6) et (R7,R8).

La polymolécularité PDI (ou dispersité *Ð*_{M}) est définie comme le rapport Mw/Mn, c'est-à-dire le rapport de la masse molaire moyenne en poids du polymère à la masse molaire moyenne en nombre dudit polymère.

Les deux masses molaires moyennes Mn et Mw sont mesurées selon l'invention par chromatographie d'exclusion stérique (ou SEC, acronyme de « Size Exclusion Chromatography » en anglais), usuellement avec étalonnage PEG (PolyEthylèneGlycol) ou PS (PolyStyrène), de préférence PS.

De préférence les groupes R5 à R8 sont chacun un atome d'hydrogène.

Si p1 = 0 ou p2 = 0, alors il n'y a pas de groupe divalent (CH₂)ₚ₁ ou (CH₂)_{P2} dans la formule F1 ou F2 et -(CH₂)ₚ₁- devient une liaison simple -ou -(CH₂)ₚ₂- devient une liaison simple - .

Lorsque l'indice m, n ou n-1, x ou y, qui s'applique à un ensemble de deux crochets est égal à zéro, cela signifie qu'il n'y a pas de groupe entre les crochets auxquels cet indice s'applique. Ainsi, 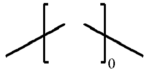 signifie -, et 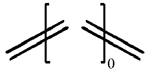 signifie =.

Chacune des doubles liaisons du polymère de formule (1) est orientée géométriquement cis ou trans, et est de préférence d'orientation cis. Les isomères géométriques du polymère de formule (1) sont généralement présents en proportions variables, avec le plus souvent une majorité de cis (Z) et préférentiellement toutes les doubles liaisons sont orientées cis (Z). Il est aussi possible selon l'invention d'obtenir un seul des isomères géométriques, selon les conditions réactionnelles et en particulier selon la nature du catalyseur utilisé.

Les polymères de formule (1) selon l'invention peuvent être des polymères solides ou des polymères liquides, à température ambiante (environ 23°C).

De préférence, ce sont des polymères liquides à 23°C, c'est-à-dire ayant une viscosité allant de 1 à 500 000 mPa.s à 23°C.

De manière générale, la viscosité peut être mesurée de manière bien connue de l'homme du métier. En particulier, la viscosité peut être mesurée avec un viscosimètre Brookfield, en choisissant l'aiguille et la vitesse du module de manière appropriée, en fonction de la gamme de viscosité à mesurer.

Selon une variante de l'invention, lorsque m est égal à 0, le polymère de formule (1) peut être représenté par la formule (2) suivante : 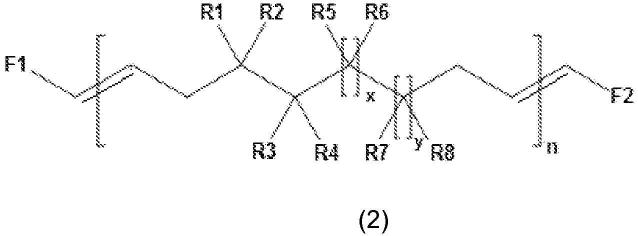 dans laquelle : x, y, n, F1, F2, R1, R2, R3, R4, R5, R6, R7 et R8 ont les significations données précédemment.

La formule (2) illustre le cas où la chaîne principale du polymère de formule (1) comporte un seul type d'unité de répétition, répétée n fois.

De façon particulièrement préférée, x est égal à 1 et y est égal à 1.

Lorsque selon une variante préférée, m est égal à 0 et R1 à R8 désignent des atomes d'hydrogène, avec au moins une des paires (R1,R2), (R3,R4), (R5, R6) et/ou (R7,R8) pouvant désigner un groupe oxo, le polymère de formule (2) est solide à température ambiante.

Selon une autre variante de l'invention, de préférence :
- m est différent de zéro
   et/ou
- l'un au moins des groupes R1 à R8 et/ou R13 à R16 comporte un groupement alkyle (et donc désigne un groupe différent d'un atome d'hydrogène et d'un atome d'halogène)
   ou
- au moins un des groupes R1 à R8 fait partie d'un même cycle hydrocarboné ou hétérocycle, saturé ou insaturé, avec au moins un autre des groupes R1 à R8, selon les règles de valence de la chimie organique,
   avec
- au moins une des paires (R1,R2), (R3,R4), (R5,R6) et/ou (R7,R8) pouvant désigner un groupe oxo.

Dans ce cas, le polymère de formule (1) est liquide à température ambiante.

Selon un mode de réalisation particulier de l'invention F1 et F2 sont identiques.

Selon un mode de réalisation préféré de l'invention, F1 et F2 sont différents.

Selon un mode de réalisation particulier, X est un atome d'oxygène.

Selon un autre mode de réalisation particulier, X est un groupe NR₁₂ où R₁₂ est tel que défini précédemment.

De préférence :
- p1 ou p2 = 0, et plus préférentiellement p1=p2 = 0,
- X est un atome d'oxygène ou un groupe NR12 où R12 est un groupe méthyle,
- A est un groupe alkylène en C1-C6,
- R9 est un atome d'hydrogène,
- R10 et R11 sont des groupes méthyle.

Les polymères hydrocarbonés à terminaisons exo-vinylène cyclocarbonate selon l'invention peuvent former, après réaction à des températures inférieures à 100°C avec des amines ou oligomères d'amines, un joint adhésif qui présente des valeurs de cohésion élevées, en particulier supérieures à 1,5 MPa (mégapascal). De telles valeurs de cohésion permettent une utilisation comme adhésif, par exemple dans le domaine de la construction, du transport ou de l'industrie en général.

L'aptitude qu'ont les polymères hydrocarbonés à terminaisons exovinylène cyclocarbonate selon l'invention à réagir plus rapidement comparativement à ceux de l'art antérieur avec des amines ou oligomères d'amines à des températures inférieures à 100°C, voire à température ambiante (c'est-à-dire environ 23°C) pour les polymères liquides de l'invention, est donc particulièrement avantageuse.

Les polymères hydrocarbonés à terminaisons exo-vinylène cyclocarbonate selon l'invention permettent ainsi d'obtenir un joint adhésif avec des propriétés mécaniques satisfaisantes, plus rapidement comparativement aux polymères hydrocarbonés de l'art antérieur cité précédemment.

Les propriétés mécaniques du joint adhésif, et notamment ses valeurs de cohésion, peuvent être mesurées conformément à la norme ISO 527-1 ou ASTM D 638-03, et notamment dans les conditions opératoires décrites dans l'exemple 9 de la demande de brevet FR No. 15/50500. Dans cet exemple, la mesure de la résistance et de l'allongement à la rupture par essai de traction a été effectuée, pour chacune des compositions adhésives, selon le protocole décrit ci-après.

Le principe de la mesure de la résistance et de l'allongement à la rupture des compositions adhésives par essai de traction consiste à étirer dans une machine de traction, dont la mâchoire mobile se déplace à une vitesse constante égale à 100 mm/min, une éprouvette standard constituée de la composition adhésive réticulée et à enregistrer, au moment où se produit la rupture de l'éprouvette, la contrainte de traction appliquée (en MPa) ainsi que l'allongement de l'éprouvette (en %).

L'éprouvette standard est en forme d'haltère, comme illustré dans la norme internationale ISO 37. La partie étroite de l'haltère utilisée a pour longueur 20 mm, pour largeur 4 mm et pour épaisseur 500 µm.

Pour préparer l'haltère, la composition adhésive a été chauffée à 100°C, puis extrudée sur une feuille A4 de papier siliconé en une quantité suffisante pour former sur celle-ci un film ayant une épaisseur de 300 µm. Le film est laissé durant 7 jours à 23 °C et 50% d'humidité relative pour réticulation, puis l'haltère est obtenue par simple découpe dans le film réticulé.

L'invention concerne encore un procédé de préparation d'au moins un polymère hydrocarboné comprenant deux groupements terminaux exo-vinylène cyclocarbonate selon l'invention, ledit procédé comprenant au moins une étape de polymérisation par ouverture de cycle par métathèse (ou « Ring-Opening Metathesis Polymerization» en anglais), en présence :
- d'au moins un catalyseur de métathèse, de préférence un catalyseur comprenant du ruthénium, de façon encore plus préférée un catalyseur de Grubbs,
- d'au moins un agent de transfert de chaîne (CTA pour « Chain Transfer Agent » en anglais) mono ou di-exo vinylène cyclocarbonate respectivement de formule (C1) ou (C2) suivante : 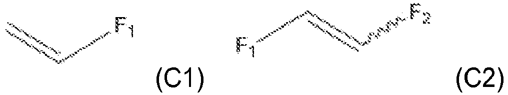 dans lesquelles :
   - F1 et F2 sont tels que définis précédemment, et
   - la liaison 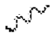 est une liaison simple carbone-carbone orientée géométriquement d'un côté ou de l'autre par rapport à la double liaison (cis ou trans) dans la formule (C2) ;
- d'au moins un composé de formule (A) suivante : 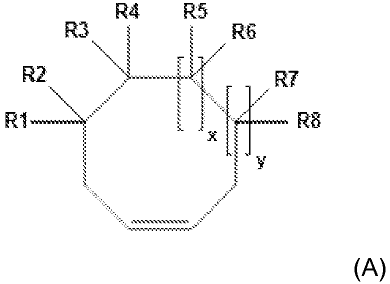 dans laquelle :
   - les groupes R1, R2, R3, R4, R5, R6, R7 et R8, identiques ou différents, sont choisis parmi un atome d'hydrogène, un atome d'halogène, un groupe alkyle, un groupe hétéroalkyle, un groupe alcoxycarbonyle ou un groupe hétéroalcoxycarbonyle,
   - au moins un des groupes R1 à R8 pouvant faire partie d'un même cycle hydrocarboné ou hétérocycle, saturé ou insaturé, avec au moins un autre des groupes R1 à R8, selon les règles de valence de la chimie organique, et
   - au moins une des paires (R1 ,R2), (R3,R4), (R5,R6) et (R7,R8) pouvant être un groupe oxo ;
   - x et y sont des nombres entiers indépendamment compris dans une fourchette allant de 0 à 5, de préférence allant de 0 à 2, de façon encore plus préférée x est égal à 1 et y est égal à 1, la somme x + y étant de préférence comprise dans une fourchette allant de 0 à 4 et de façon encore plus préférée allant de 0 à 2 ;
      et
- d'éventuellement au moins un composé de formule (B) : 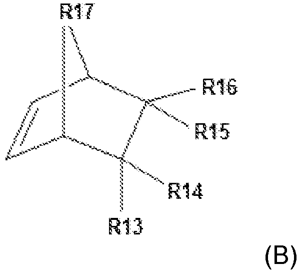 dans laquelle :
   - les groupes R13, R14, R15 et R16, identiques ou différents, sont choisis parmi un atome d'hydrogène, un atome d'halogène, un groupe hétéroalkyle, un groupe alcoxycarbonyle et un groupe hétéroalcoxycarbonyle,
   - au moins un, et de préférence un, des groupes R13 à R16 pouvant faire partie d'un même cycle ou hétérocycle saturé ou insaturé avec au moins un autre, et de préférence avec un autre, des groupes R13 à R16, selon les règles de valence de la chimie organique ; et
   - le groupe R17 est CH2, O, S, C(=O) ou NR₀, R₀ étant un groupe alkyle, de préférence linéaire, comportant de 1 à 22, de préférence de 1 à 14, atomes de carbone ;
   pendant une durée de réaction allant de 2 à 24 heures et à une température comprise dans une fourchette allant de 20 à 60°C.

La durée et la température pour cette réaction de polymérisation dépendent généralement des conditions de réaction et en particulier du taux de charge catalytique. L'homme du métier est à même de les adapter en fonction des circonstances.

Le CTA est un composé qui comprend une ou deux fonctions exovinylène cyclocarbonate.

Le CTA de formule (C1) est dit monofonctionnel lorsqu'il comporte un groupement exo-vinylène cyclocarbonate.

Le CTA de formule (C2) est dit difonctionnel lorsqu'il comporte deux groupements exo-vinylène cyclocarbonate, pouvant être identiques ou différents.

Le rapport molaire du CTA de formule (C1) sur le composé de formule (A), ou sur la somme des composés de formules (A) et (B) si le composé de formule (B) est présent, est compris dans une fourchette allant de 1.10⁻³ à 1,0 et de préférence allant de 1.10⁻² à 0,250.

Le rapport molaire du CTA de formule (C2) sur le composé de formule (A), ou sur la somme des composés de formules (A) et (B) si le composé de formule (B) est présent, est compris dans une fourchette allant de 0,5.10⁻³ à 0,5 et de préférence allant de 0,5.10⁻² à 0,125.

Les composés de formule (A) comprennent généralement de 6 à 30, de préférence de 6 à 22, atomes de carbone.

Les composés de formule (B) comprennent généralement de 6 à 30, de préférence de 6 à 22, atomes de carbone.

Dans un mode de réalisation préféré de l'invention, x = y = 1.

La polymérisation par ouverture de cycle par métathèse (ou ROMP pour « Ring-Opening Methathesis Polymerization » en anglais) est une réaction bien connue de l'homme du métier. Dans la présente demande, cette réaction est mise en oeuvre en présence d'un CTA mono-fonctionnel de formule (C1) ou d'un CTA difonctionnel de formule (C2).

Les composés cycliques de formule (A) sont de préférence, selon l'invention, choisis dans le groupe formé par le cycloheptène, le cyclooctène, le cyclononène, le cyclodécène, le cycloundécène, le cyclododécène, le 1,5-cyclooctadiène, le cyclononadiène, le 1,5,9-cyclodécatriène, le 5-époxycyclooctène, le 5-oxocyclooctène, les 5-alkyl-cyclooctène dont la partie alkyle en C1-C22, et préférence en C1-C14, et leurs mélanges. Le cyclooctène (COE) 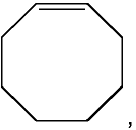 le 5-époxy-cyclooctène 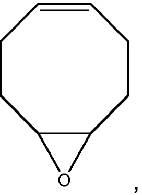 le 5-oxocyclooctène 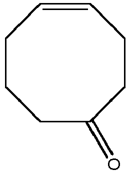 et les 5-alkyl-cyclooctène 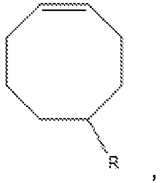 où R est un groupe alkyle comprenant de 1 à 22, de préférence 1 à 14, atomes de carbone, sont préférés selon l'invention ; le cyclooctène étant tout particulièrement préféré. Par exemple, R est un groupe n-hexyle.

Les composés cycliques de formule (B) sont de préférence selon l'invention choisis dans le groupe formé par les norbornène (NBN), dicyclopentadiène et 7-oxanorbornène qui sont respectivement de formules suivantes :

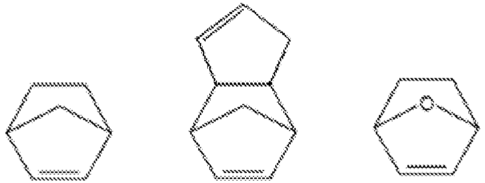

Les composés cycliques de formule (B) peuvent également être choisis dans le groupe formé par les composés de formules suivantes : 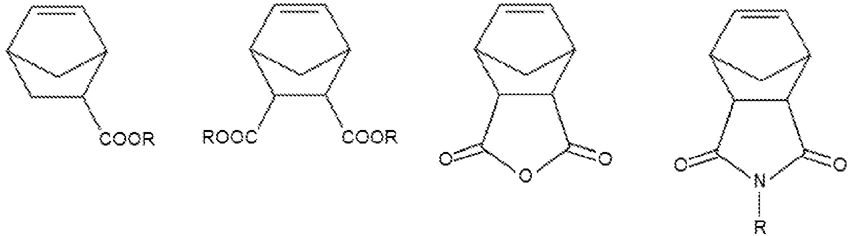 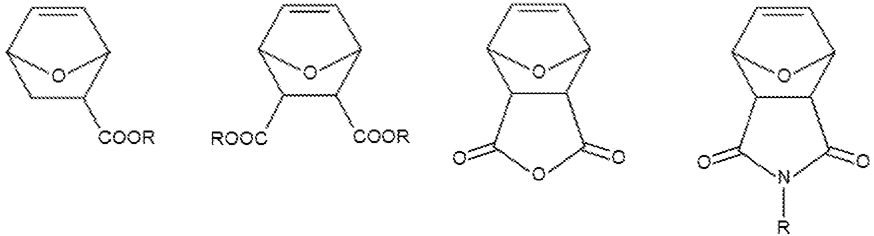 dans lesquelles : R est un groupe alkyl comprenant de 1 à 22, de préférence 1 à 14, atomes de carbone. Par exemple, R est un groupe n-hexyle.

Les composés cycliques de formule (B) peuvent également être choisis dans le groupe formé par les produits d'addition (ou adducts en anglais) issus de la réaction de Diels-Alder utilisant le cyclopentadiène ou le furane comme produit de départ, ainsi que les composés dérivés du norbornène tels que décrits dans WO 2001/04173 (tels que : carboxylate d'isobornyl norbornène, carboxylate de phényl norbornène, carboxylate d'éthylhéxyle norbornène, carboxylate de phénoxyéthyle norbornène et alkyl dicarboxymide norbornène, l'alkyl comportant le plus souvent de 3 à 8 atomes de carbone) et ceux décrits dans WO 2011/038057 (anhydrides dicarboxyliques de norbornène et éventuellement anhydrides dicarboxyliques de 7-oxanorbornène).

Selon un premier mode de réalisation du procédé de préparation selon l'invention (appelé « voie CTA monofonctionnel »), le CTA utilisé est de formule (C1) définie précédemment et pouvant également être représentée comme suit, avec p1, X, A, R9, R10 et R11 ayant les significations données précédemment : 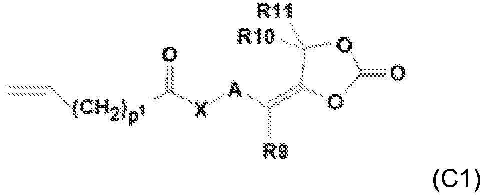 Dans ce cas, de préférence,
- p1 = 0,
- X est un atome d'oxygène ou un groupe NR12 où R12 est un groupe méthyle,
- A est un groupe alkylène en C1-C6,
- R9 est un atome d'hydrogène,
- R10 et R11 sont des groupes méthyle.

Le composé de formule (C1) peut être obtenu selon le schéma (1) ci-dessous, en suivant les modes opératoires décrits dans les demandes de brevet DE1098953 et DE3433403, p1, X, A, R9, R10 et R11 ayant les significations données précédemment :

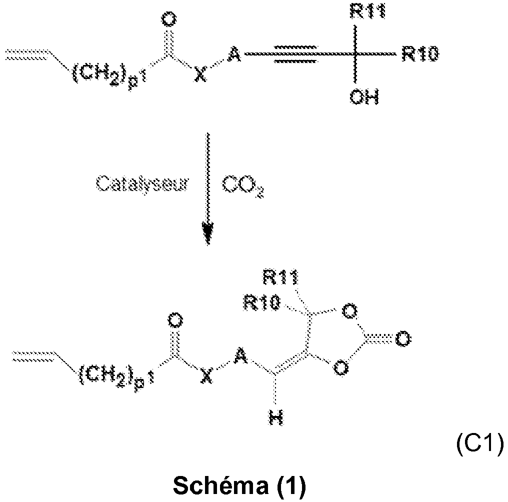

Selon un second mode de réalisation du procédé de préparation selon l'invention (appelé « voie CTA difonctionnel »), le CTA2 est de formule (C2) définie précédemment et pouvant également être représentée comme suit, avec p1, p2, Z, X, A, R9, R10, R11 et 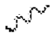 ayant les significations données précédemment : 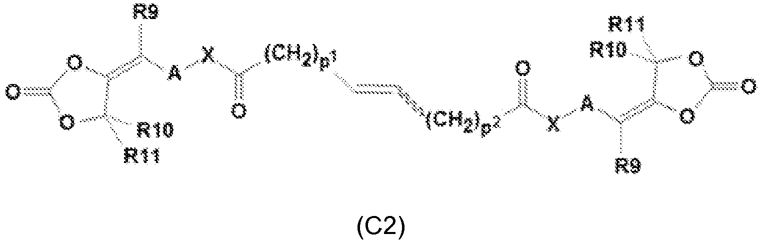 Dans ce cas, de préférence,
- p1 ou p2 = 0, et plus préférentiellement p1=p2=0,
- X est un atome d'oxygène ou un groupe NR12 où R12 est un groupe méthyle,
- A est un groupe alkylène en C1-C6,
- R9 est un atome d'hydrogène,
- R10 et R11 sont des groupes méthyle.

Le composé de formule (C2) peut être obtenu selon le schéma (2) ci-dessous, correspondant à une variante originale du schéma (1), p1, p2, X, A, R9, R10 et R11 ayant les significations données précédemment :

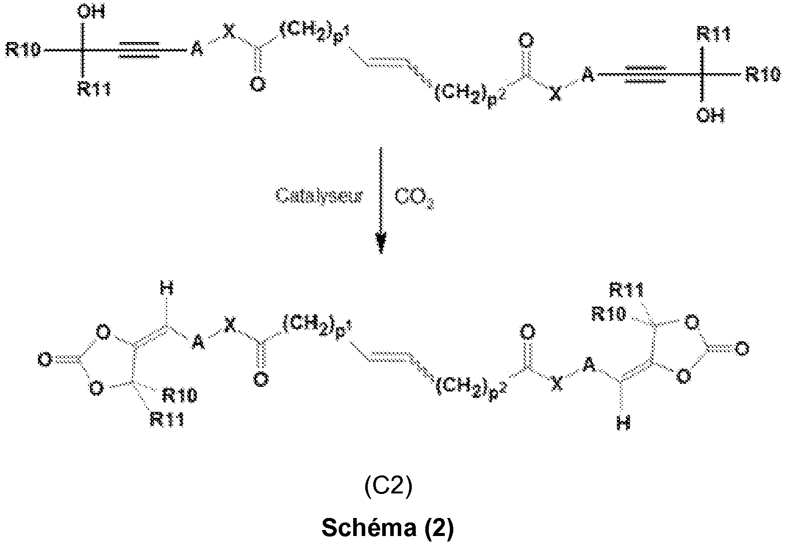

L'étape de polymérisation par ouverture de cycle par métathèse est mise en oeuvre le plus souvent en présence d'au moins un solvant, généralement choisi dans le groupe formé par les solvants aqueux ou organiques typiquement utilisés dans les réactions de polymérisation et qui sont inertes dans les conditions de la polymérisation sus-décrites.

A titre d'exemple de solvant pouvant être mis en oeuvre, on peut citer par exemple, les hydrocarbures aromatiques, les hydrocarbures chlorés, les éthers, les hydrocarbures aliphatiques, les alcools, l'eau ou leurs mélanges.

De préférence, le solvant est choisi dans le groupe formé par le benzène, le toluène, le para-xylène, le chlorure de méthylène, le dichloroéthane, le dichlorobenzène, le chlorobenzène, le tétrahydrofurane, le diéthyl éther, le pentane, l'hexane, l'heptane, le méthanol, l'éthanol, l'eau ou leurs mélanges.

Plus préférentiellement, le solvant est choisi dans le groupe formé par le benzène, le toluène, le para-xylène, le chlorure de méthylène, le dichloroéthane, le dichlorobenzène, le chlorobenzène, le tétrahydrofurane, le diéthyl éther, le pentane, l'hexane, l'heptane, le méthanol, l'éthanol, et leurs mélanges.

Mieux encore, le solvant est le toluène, l'heptane, ou un mélange de toluène et de chlorure de méthylène. La solubilité du polymère formé au cours de la réaction de polymérisation dépend généralement et principalement du choix du solvant et de la masse molaire du polymère obtenu. Il est aussi possible que la réaction soit mise en oeuvre sans solvant.

Le catalyseur de métathèse, tel que par exemple un catalyseur de Grubbs, est généralement un produit du commerce.

Le catalyseur de métathèse est le plus souvent un catalyseur comprenant au moins un métal de transition, tel que le ruthénium, le plus souvent sous la forme de complexe.

De préférence, le catalyseur de métathèse est choisi parmi les complexes du ruthénium tel qu'un complexe ruthénium-carbène.

Plus préférentiellement, le catalyseur de métathèse est choisi parmi les catalyseurs de Grubbs.

Par « catalyseur de Grubbs », on entend généralement selon l'invention un catalyseur de Grubbs 1^{ère} ou 2^{ème} génération, mais aussi tout autre catalyseur de type Grubbs (de type ruthénium-carbène) accessible à l'homme du métier, tel que par exemple les catalyseurs de Grubbs substitués décrits dans le brevet US 5,849,851.

Un catalyseur de Grubbs 1^{ère} génération est généralement de formule (G1): 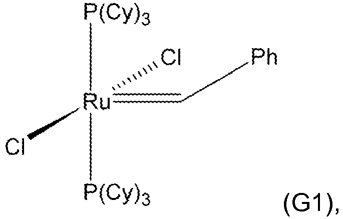 dans laquelle :
- Ph est le groupe phényle
- Cy est le groupe cyclohexyle, et
- P(Cy)₃ est un groupe tricyclohexylphosphine.

La dénomination IUPAC du catalyseur (G1) est: benzylidène-bis(tricyclohexylphosphine) dichlororuthénium (de numéro CAS 172222-30-9).

Un catalyseur de Grubbs 2^{ème} génération est généralement de formule (G2) : 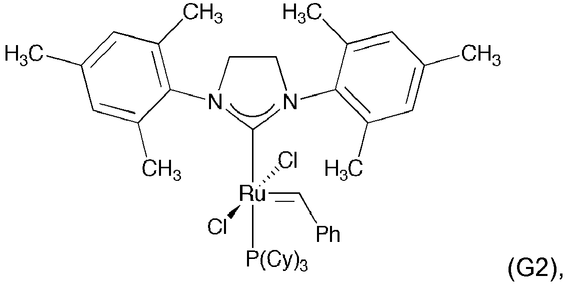 dans laquelle :
- Ph est le groupe phényle,
- Cy est le groupe cyclohexyle, et
- P(Cy)₃ est un groupe tricyclohexylphosphine.

La dénomination IUPAC du catalyseur (G2) est : benzylidène [1,3-bis(2,4,6-triméthylphényl)-2-imidazolidinylidène]dichloro(tricyclohexylphosphine) ruthénium (de numéro CAS 246047-72-3).

De préférence, le catalyseur est un catalyseur de Grubbs 2^{ème} génération, tel que défini par exemple par la formule (G2).

L'invention concerne également un procédé de préparation de polyuréthane comprenant la réaction d'au moins un polymère hydrocarboné à terminaisons exo-vinylène cyclocarbonate selon l'invention, susceptible notamment d'être obtenu par le procédé de préparation selon l'invention, avec au moins un composé comprenant au moins un, de préférence au moins deux, groupements aminés, par exemple choisi parmi les monoamines, les diamines, les triamines et les autres polyamines ; ainsi que les polyuréthanes susceptibles d'être obtenus par ce procédé de préparation.

Les composés aminés sus-décrits sont de préférence telles qu'au moins un groupement amine, de préférence tous les groupements amines, sont des groupements amines primaires. Ces composés aminés peuvent être des oligomères. Ces oligomères ont généralement une masse molaire moyenne en nombre inférieure à 2000.

De préférence, les polymère(s) hydrocarboné(s) à terminaisons exovinylène cyclocarbonate et composé(s) aminés sont utilisés en quantités telles que l'ensemble des groupes exo-vinylène cyclocarbonate du (des) polymère(s) ont réagi avec un groupe amine d'un composé aminé pour former un groupe uréthane.

Plus préférentiellement, les polymère(s) hydrocarboné(s) à terminaisons exo-vinylène cyclocarbonate sont mis à réagir avec un ou plusieurs (poly)amine(s) primaire(s) en quantités stoechiométriques, c'est-à-dire que le rapport molaire du nombre de groupes cyclocarbonates sur le nombre de groupes amines primaires est environ égal à 1.

Les polyuréthanes ainsi obtenus, qui sont nouveaux, ont été avantageusement préparés sans isocyanate.

Ces polyuréthanes, une fois formulés (c'est-à-dire mis en formulation avec d'autres additifs éventuels), sont destinés à être utilisés en tant que tels comme compositions de revêtements, de mastics ou d'adhésifs, ou dans des compositions de revêtements, de mastics ou d'adhésifs, par exemple en tant que charges et/ou en tant que résines. Il est aussi possible de formuler indépendamment le polymère à terminaisons exo-vinylène cyclocarbonate selon l'invention et le composé comprenant au moins un groupement amine, avant leur mélange, notamment sous la forme d'une composition bi-composant.

L'invention sera mieux comprise à la vue des exemples qui suivent.

### EXEMPLES

Les exemples qui suivent illustrent l'invention sans pour autant en limiter la portée.

### I - Exemples 1 à 9 : Synthèse d'un polymère à terminaisons exo-vinylène cyclocarbonate selon l'invention

Les polymères hydrocarbonés à terminaisons exo-vinylène cyclocarbonate des exemples 1 à 9 ont été obtenus à l'aide des étapes suivantes :
1- une étape de synthèse de(s) cyclooléfine(s) de formules (A) et/ou (B),
2- une étape de synthèse de l'agent de transfert (CTA) de formule (C1) ou (C2),
3- une étape de polymérisation par ouverture de cycle par métathèse de cyclooléfine de formule (A) et éventuellement de composé de formule (B) en présence d'un catalyseur de Grubbs et de l'agent de transfert, l'étape 1 étant de préférence facultative lorsque les cyclooléfine(s) des formules (A) et (B) sont disponibles dans le commerce.

Les réactions de polymérisation par ouverture de cycle par métathèse mises en oeuvre dans les exemples 1 à 9 sont représentées par les schémas généraux (3) et (4), utilisant respectivement un CTA monofonctionnel (C1) et un CTA difonctionnel (C2), et seront explicités au cas par cas dans les exemples. 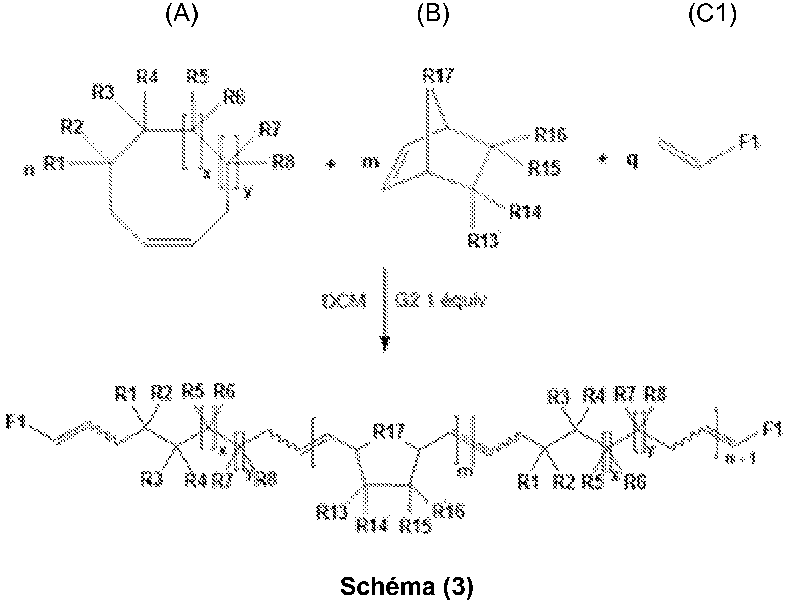 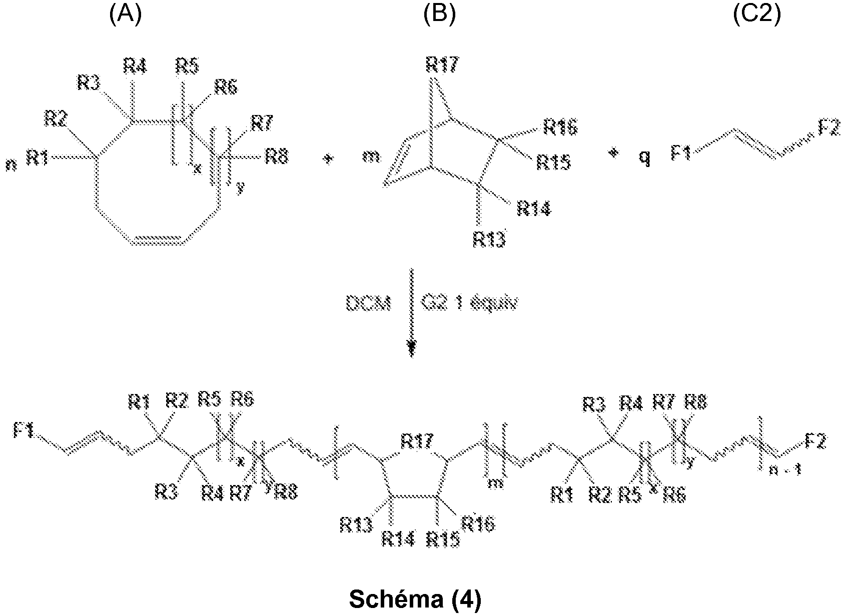 Dans ces schémas (3) et (4) :
- 1 équiv. signifie un équivalent et correspond à la quantité de catalyseur de métathèse utilisée ;
- DCM signifie dichlorométhane ;
- (A) et (B) sont les cyclooléfines répondant respectivement aux formules (A) et (B) définies précédemment ;
- (C1) et (C2) sont les agents de transfert répondant respectivement aux formules (C1) et (C2) définis précédemment ;
- la liaison 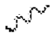 une liaison simple carbone-carbone orientée géométriquement d'un côté ou de l'autre par rapport à la double liaison (cis ou trans) pour (C2) ;
- G2 est le catalyseur de métathèse de formule (G2) telle que définie précédemment ;
- F1 et F2 sont identiques et correspondent tous deux :
   soit au groupe de type ester ci-dessous : 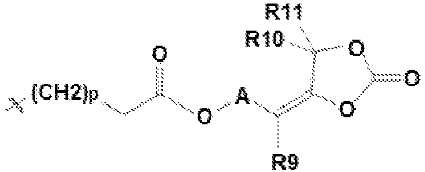
   soit au groupe de type amide : 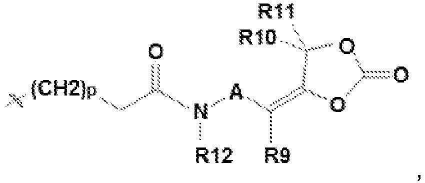 dans lesquels : p = p1 ou p2, et A, R9, R10 et R11 étant tels que définis précédemment ;
- n est le nombre de moles de cyclooléfine(s) de formule (A) ;
- m est le nombre de moles de cyclooléfine(s) de formule (B) ;
- q est le nombre de moles de CTA de formule (C1) ou (C2).

Le nombre d'unités monomères dans le polymère obtenu à l'issue de la réaction de polymérisation est égal à n + m.

Dans chacun des exemples 1 à 9 décrits ci-après, la réaction dure 24heures (h) à une température de 40°C.

Toutes les polymérisations ont été effectuées de façon similaire. Les seules différences résident dans la nature et la concentration initiale du (ou des) agent(s) de transfert de chaîne (CTA) de type (C1) ou (C2) utilisé(s).

Les CTA utilisés dans les exemples 1 à 9 sont les suivants:
- le [(5,5-diméthyl-2-oxo-1,3-dioxolan-4-ylidène)propyl] acrylate (noté CTA¹) 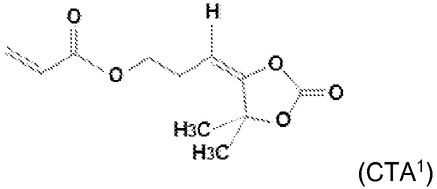 (qui correspond à un CTA de formule (C1) où : p1 = 0, X est un atome d'oxygène, A est un groupe éthylène -CH2-CH2-, R9 est un atome d'hydrogène, R10 et R11 sont des groupes méthyle) ;
- la [N-méthyl,(5,5-diméthyl-2-oxo-1,3-dioxolan-4-ylidène)propyl] acrylamide (noté CTA²) 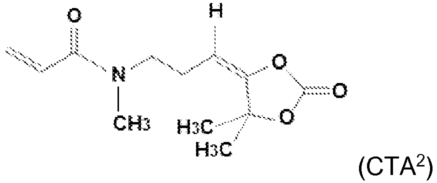 (qui correspond à un CTA de formule (C1) où p1 = 0, X est un groupe N-CH3 avec R12 étant le groupe méthyle, A est un groupe éthylène -CH2-CH2-, R9 est un atome d'hydrogène, R10 est un groupe méthyle, R11 et R12 sont des groupes méthyle) ;
- le bis[(5,5-diméthyl-2-oxo-1,3-dioxolan-4-ylidène)propyl] fumarate (noté CTA³) 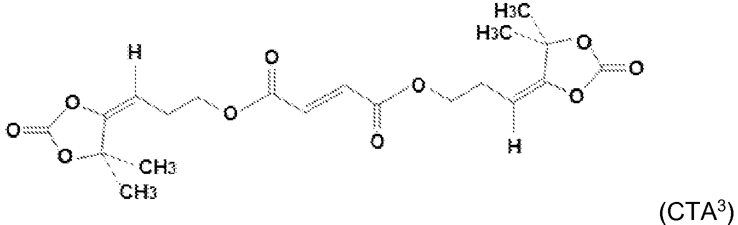 (qui correspond à un CTA de formule (C2) où : p1 = p2 = 0, X est un atome d'oxygène, A est un groupe éthylène -CH2-CH2-, R9 est un atome d'hydrogène, R10 et R11 sont des groupes méthyle) ; et
- la bis[(5,5-diméthyl-2-oxo-1,3-dioxolan-4-ylidène)propyl] fumaramide (noté CTA⁴) 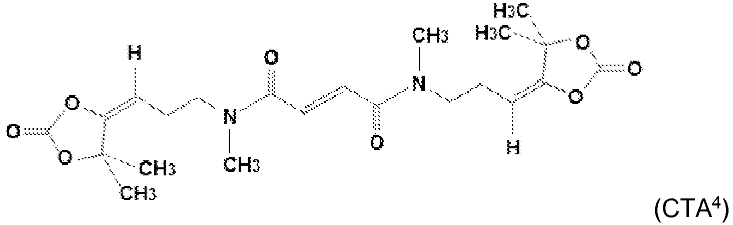 (qui correspond à un CTA de formule (C2) où : p1 = p2 = 0, X est un groupe N-CH3 avec R12 étant le groupe méthyle, A est un groupe éthylène -CH2-CH2-, R9 est un atome d'hydrogène, R10 et R11 sont des groupes méthyle).

Deux possibilités de réaction (i et ii) existent, selon que l'on utilise la cyclooléfine de formule (A) seule (exemples 1 à 7) ou selon que l'on utilise les cyclooléfines de formules (A) et (B) en mélange (exemples 8 et 9).

### i) Exemples 1 à 7 : Polymérisation des cyclooléfines de formule (A)

Les cyclooléfines de formule (A) utilisées dans les exemples 1 à 7 sont les suivantes :

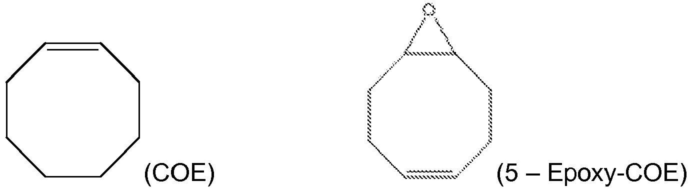

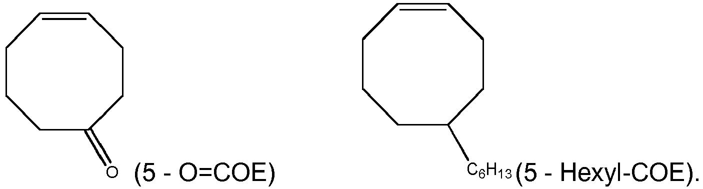

Le cyclooctène (COE) et le 5,6 époxycyclooctène (5-EpoxyCOE) sont des produits commerciaux de la société Sigma Aldrich.

Le 5-oxocyclooctène (5-O=COE) et le 5-*n*-hexyl-cyclooctène (5-Hexyl-COE) peuvent être synthétisés à partir du 5,6-époxycyclooctène (5-Epoxy-COE) selon la voie indiquée dans le schéma réactionnel (5) suivant :

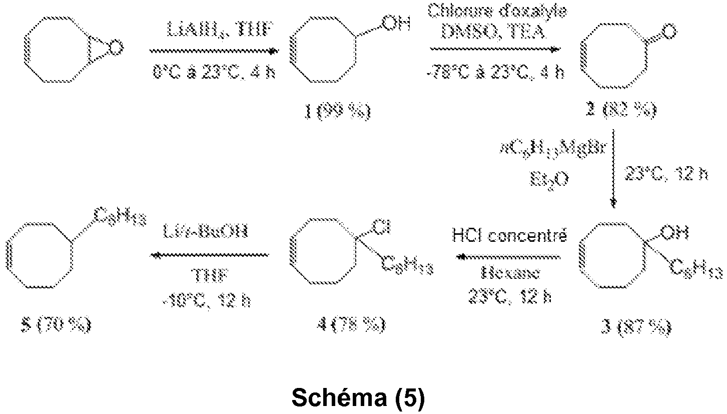

Le 5-oxocyclooctène (5-O=COE, référencé 2 dans le schéma (5) ci-dessus) a été synthétisé selon la procédure indiquée dans la publication de A.Diallo et al., Polymer Chemistry, Vol. 5, Issue 7, 7 April 2014, pages 2583-2591 (qui se référait à Hillmyer et al, Macromolecules, 1995, 28, pages 6311-6316).

Le 5-hexyle-cyclooctène (5-Hexyl-COE référencé 5 dans le schéma (5) ci-dessus) a été synthétisé selon la procédure indiquée dans la publication de A.Diallo et al., Polymer Chemistry, précitée (qui se référait à Kobayashi et al., J. Am. Chem. Soc. 2011, 133, pages 5794-5797).

Les matières premières, réactifs et solvants utilisés pour la synthèse de ces cyclooléfines de formule (A) sont disponibles commercialement auprès de la société Sigma Aldrich.

Dans les exemples qui suivent :
- Les spectres RMN ont été enregistrés sous spectromètres AM-500 Bruker et AM-400 Brüker, à 298 K dans CDCl₃. Les déplacements chimiques étaient référencés par rapport au tétramethylsilane (TMS) en utilisant la résonance (¹H) ou (¹³C) des solvants deutérés.
- Les masses molaires moyennes en nombre et en poids (Mₙ and M_{w}) et la polydispersité PDI (M_{w}/Mₙ) des polymères ont été déterminés par chromatographie d'exclusion stérique (SEC), avec étalonnage PS en utilisant un instrument Polymer Laboratories PL-GPC 50.

### Exemple 1 : Synthèse d'un polymère comprenant deux groupements terminaux [(5,5-diméthyl-2-oxo-1,3-dioxolan-4-ylidène)propyl] ester à partir de cyclooctène (COE) et de [(5,5-diméthyl-2-oxo-1,3-dioxolan-4-ylidène)propyl] acrylate (CTA¹)

La réaction a été mise en oeuvre selon le schéma (6) suivant, dans un rapport molaire m/n égal à 0/100 et selon le mode opératoire décrit ci-après :

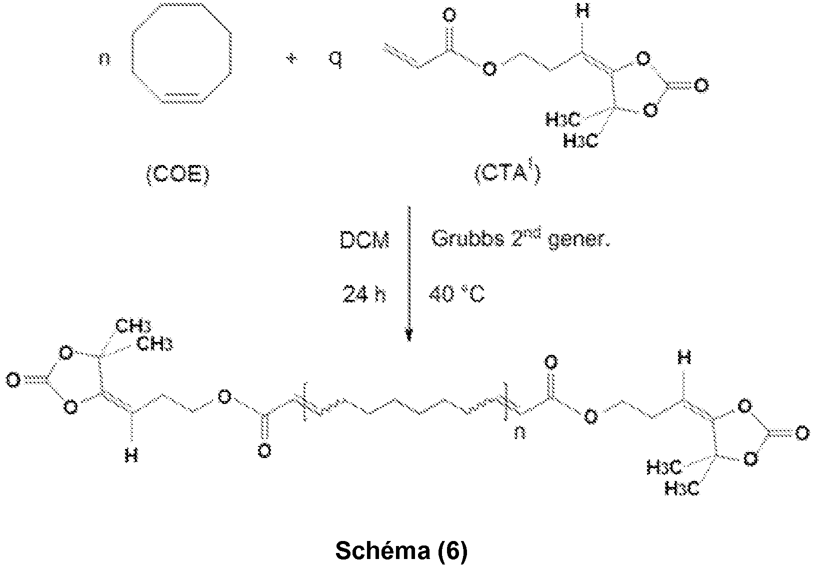

### Mode opératoire :

On a mélangé dans un ballon de 1000 mL, la cyclooléfine de formule (A) (*108,00 mmol*), de l'hydroquinone (*0,54 mmole*) et du CH₂Cl₂ sec (50 mL). Le ballon et son contenu ont ensuite été mis sous argon. Le CTA (*10,80 mmol*) de type (C1) a été introduit dans le ballon par seringue. Le ballon a alors été immergé dans un bain d'huile à 40 °C puis on a procédé immédiatement à l'ajout, par une canule, du catalyseur G2 (*54,00 µmol*) en solution dans du CH₂Cl₂ (20 mL). 24 h après l'addition du catalyseur, le produit est extrait du ballon après évaporation du solvant sous vide. Le produit est ensuite récupéré après précipitation dans le méthanol, filtration et séchage à 20 °C sous vide.

### Résultats :

Le polymère obtenu est solide à température ambiante.

Le taux de conversion de la cyclooléfine de formule (A) déterminé par RMN (exprimé en %), la masse molaire moyenne en nombre du polymère obtenu (exprimé en gramme par mol) ainsi que la polymolécularité (PDI) dudit polymère, déterminés par SEC, ont donné les résultats suivants :

**Tableau 1 :**

| **Essai N°** | **[A]/[CTA¹]/[Ru] (mol/mol)** | **Conversion A (%)** | **Mn_{SEC} (g/mol)** | **PSI** |
|---|---|---|---|---|
| **1** | 2 000/200/1 | 100 | 4 600 | 1,53 |

Les analyses RMN ¹H (CDCl₃, 500 MHz, 298 K) et ¹³C (CDCl₃, 100 MHz, 298 K) du polymère obtenu pour cet essai ont confirmé la structure du polymère attendu tel que représenté sur le schéma (6).

### Exemple 2 : Synthèse d'un polymère comprenant deux groupements terminaux [N-méthyl,(5,5-diméthyl-2-oxo-1,3-dioxolan-4-ylidène)propyl] amide à partir de cyclooctène (COE) et de [N-méthyl,(5,5-diméthyl-2-oxo-1,3-dioxolan-4-ylidène)propyl] acrylamide (CTA²)

La réaction a été mise en oeuvre selon le schéma (7) suivant, dans un rapport molaire m/n égal à 0/100 et selon le mode opératoire que l'exemple 1 :

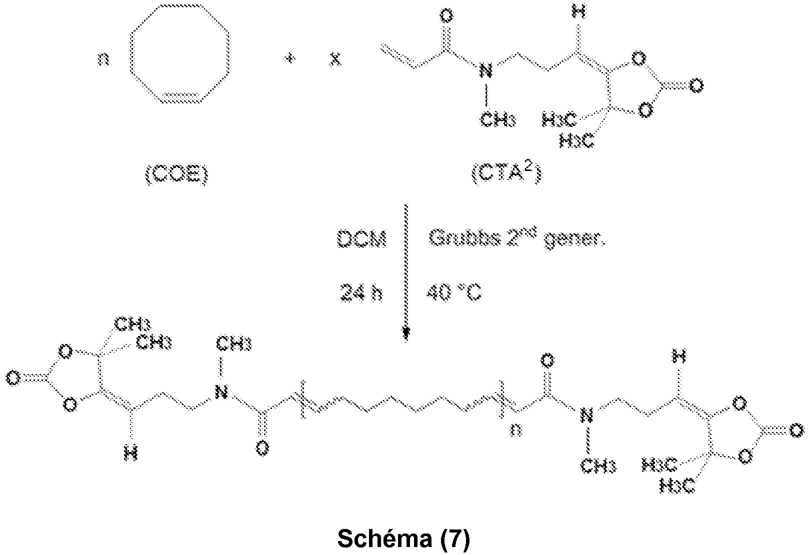

### Résultats :

Le polymère obtenu est solide à température ambiante.

Le taux de conversion de la cyclooléfine de formule (A) déterminé par RMN (exprimé en %), la masse molaire moyenne en nombre du polymère obtenu (exprimé en gramme par mol) ainsi que la polymolécularité (PDI) dudit polymère, déterminés par SEC, ont donné les résultats suivants :

**Tableau 2 :**

| **Essai n°** | **[A]/[CTA²]/[Ru](mol/mol)** | **Conversion A (%)** | **Mn_{SEC} (g/mol)** | **PDI** |
|---|---|---|---|---|
| **2** | 2 000/200/1 | 100 | 4 900 | 1,49 |

Les analyses RMN ¹H (CDCl₃, 500 MHz, 298 K) et ¹³C (CDCl₃, 100 MHz, 298 K) du polymère obtenu pour cet essai ont confirmé la structure du polymère attendu tel que représenté sur le schéma (7).

### Exemple 3 : Synthèse d'un polymère comprenant deux groupements terminaux [(5,5-diméthyl-2-oxo-1,3-dioxolan-4-ylidène)propyl] ester à partir de cyclooctène (COE) et de bis[(5,5-diméthyl-2-oxo-1,3-dioxolan-4-ylidène)propyl] fumarate (CTA³)

La réaction a été mise en oeuvre selon le schéma (8) suivant, dans un rapport molaire m/n égal à 0/100 et selon le mode opératoire décrit ci-après :

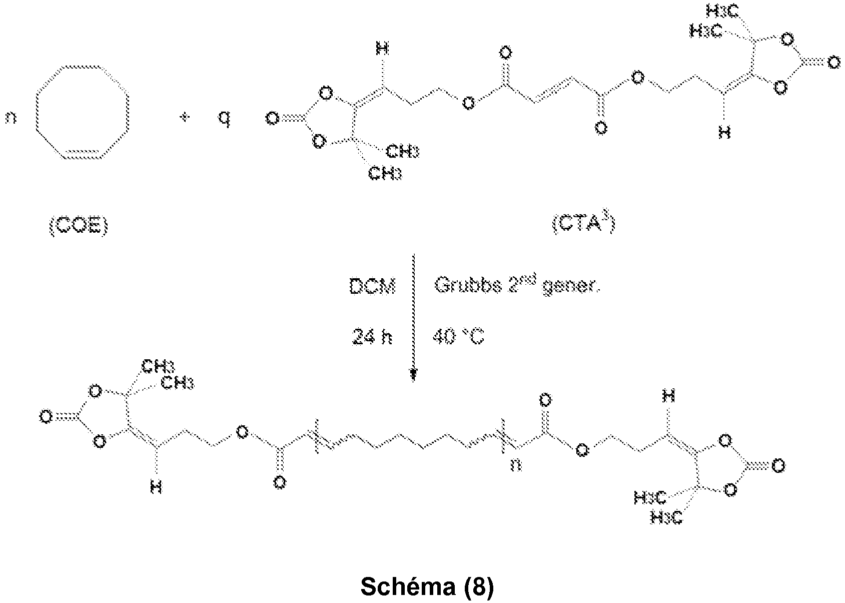

### Mode opératoire :

On mélange dans un ballon de 1000 mL, la cyclooléfine de formule (A) (*108,00 mmo*/), de l'hydroquinone (*0,54 mmole*) et du CH₂Cl₂ sec (50 mL). Le ballon et son contenu ont ensuite été mis sous argon. Le CTA (*5,40 mmol*) de type (C2) a été introduit dans le ballon par seringue. Le ballon a alors été immergé dans un bain d'huile à 40 °C puis l'on a procédé immédiatement à l'ajout, par une canule, du catalyseur G2 (*54,00 µmol*) en solution dans du CH₂Cl₂ (20 mL). 24 h après l'addition du catalyseur, le produit est extrait du ballon après évaporation du solvant sous vide. Le produit est ensuite récupéré après précipitation dans le méthanol, filtration et séchage à 20 °C sous vide.

### Résultats :

Le polymère obtenu est solide à température ambiante.

Les résultats du taux de conversion de la cyclooléfine de formule (A) déterminé par RMN (exprimé en %), de la masse molaire moyenne en nombre du polymère obtenu (exprimé en gramme par mol) ainsi que la polymolécularité (PDI) dudit polymère, déterminés par SEC, ont été consignés dans le tableau 3 plus bas.

Les analyses RMN ¹H (CDCl₃, 500 MHz, 298 K) et ¹³C (CDCl₃, 100 MHz, 298 K) du polymère obtenu pour cet essai ont confirmé la structure du polymère attendu tel que représenté sur le schéma (8).

### Exemple 4 : Synthèse d'un polymère comprenant deux groupements terminaux [(5,5-diméthyl-2-oxo-1,3-dioxolan-4-ylidène)propyl] amide à partir de cyclooctène (COE) et de bis[(5,5-diméthyl-2-oxo-1,3-dioxolan-4-ylidène)propyl] fumaramide (CTA⁴)

La réaction a été mise en oeuvre selon le schéma (9) suivant, dans un rapport molaire m/n égal à 0/100 et selon le mode opératoire que l'exemple 3 :

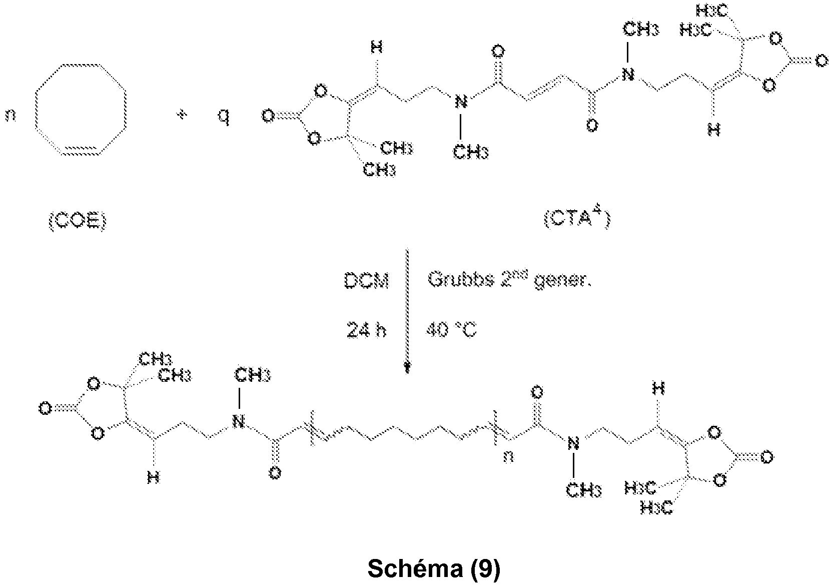

### Résultats :

Le polymère obtenu est solide à température ambiante.

Les résultats du taux de conversion de la cyclooléfine de formule (A) déterminé par RMN (exprimé en %), de la masse molaire moyenne en nombre du polymère obtenu (exprimé en gramme par mol) ainsi que de la polymolécularité (PDI) dudit polymère, déterminés par SEC, ont été consignés dans le tableau 3 plus bas.

Les analyses RMN ¹H (CDCl₃, 500 MHz, 298 K) et ¹³C (CDCl₃, 100 MHz, 298 K) du polymère obtenu pour cet essai ont confirmé la structure du polymère attendu tel que représenté sur le schéma (9).

### Exemple 5 : Synthèse d'un polymère comprenant deux groupements terminaux [(5,5-diméthyl-2-oxo-1,3-dioxolan-4-ylidène)propyl] ester à partir de cyclooctène monoépoxyde (5-Epoxy-COE) et de bis[(5,5-diméthyl-2-oxo-1,3-dioxolan-4-ylidène)propyl] fumarate (CTA³)

La réaction a été mise en oeuvre selon le schéma (10) suivant, dans un rapport molaire m/n égal à 0/100 et selon le mode opératoire que l'exemple 3 :

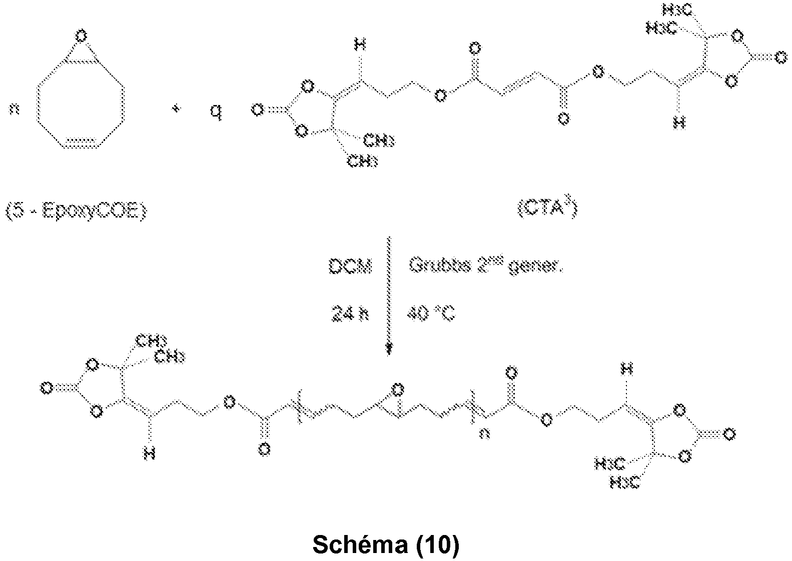

### Résultats :

Le polymère obtenu est liquide à température ambiante.

Les résultats du taux de conversion de la cyclooléfine de formule (A) déterminé par RMN (exprimé en %), de la masse molaire moyenne en nombre du polymère obtenu (exprimé en gramme par mol) ainsi que de la polymolécularité (PDI) dudit polymère, déterminés par SEC, ont été consignés dans le tableau 3 plus bas.

Les analyses RMN ¹H (CDCl₃, 500 MHz, 298 K) et ¹³C (CDCl₃, 100 MHz, 298 K) du polymère obtenu pour cet essai ont confirmé la structure du polymère attendu tel que représenté sur le schéma (10).

### Exemple 6 : Synthèse d'un polymère comprenant deux groupements terminaux [(5,5-diméthyl-2-oxo-1,3-dioxolan-4-ylidène)propyl] ester à partir de 5-oxocyclooctène (5 - O=COE) et de bis[(5,5-diméthyl-2-oxo-1,3-dioxolan-4-ylidène)propyl] fumarate (CTA³)

La réaction a été mise en oeuvre selon le schéma (11) suivant, dans un rapport molaire m/n égal à 0/100 et selon le mode opératoire que l'exemple 3 :

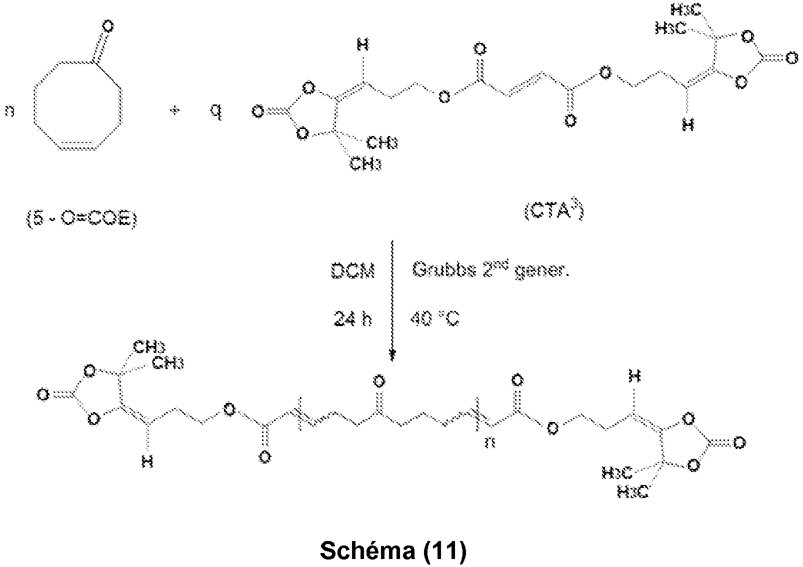

### Résultats :

Le polymère obtenu est solide à température ambiante.

Les résultats du taux de conversion de la cyclooléfine de formule (A) déterminé par RMN (exprimé en %), de la masse molaire moyenne en nombre du polymère obtenu (exprimé en gramme par mol) ainsi que de la polymolécularité (PDI) dudit polymère, déterminés par SEC, ont été consignés dans le tableau 3 plus bas.

Les analyses RMN ¹H (CDCl₃, 500 MHz, 298 K) et ¹³C (CDCl₃, 100 MHz, 298 K) du polymère obtenu pour cet essai ont confirmé la structure du polymère attendu tel que représenté sur le schéma (11).

### Exemple 7 : Synthèse d'un polymère comprenant deux groupements terminaux [(5,5-diméthyl-2-oxo-1,3-dioxolan-4-ylidène)propyl] ester à partir de 5-hexyl-cyclooctène (5-Hexyl-COE) et de bis[(5,5-diméthyl-2-oxo-1,3-dioxolan-4-ylidène)propyl] fumarate (CTA³)

La réaction a été mise en oeuvre selon le schéma (12) suivant, dans un rapport molaire m/n égal à 0/100 et selon le mode opératoire que l'exemple 3 :

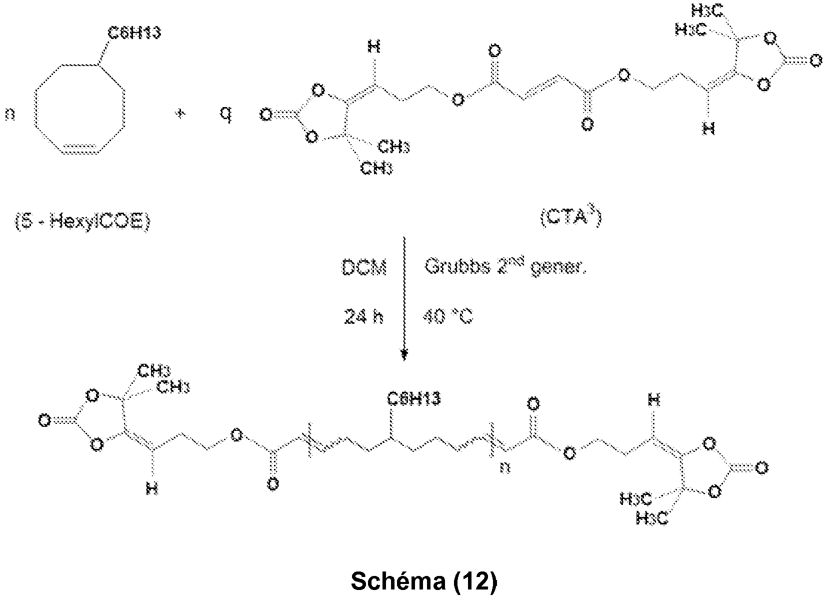

### Résultats :

Le polymère obtenu est liquide à température ambiante.

Les résultats du taux de conversion de la cyclooléfine de formule (A) déterminé par RMN (exprimé en %), de la masse molaire moyenne en nombre du polymère obtenu (exprimé en gramme par mol) ainsi que de la polymolécularité (PDI) dudit polymère, déterminés par SEC, ont été consignés dans le tableau 3 suivant :

**Tableau 3 :**

| **Essai N°** | **[A]/[CTA^{3]/}[Ru](mol/mol)** | **Conversion A (%)** | **Mn_{SEC} (g/mol)** | **PDI** |
|---|---|---|---|---|
| **3** | 2 000/100/1 | 100 | 5 100 | 1,53 |
| **4** | 2 000/100/1 | 100 | 5 200 | 1,47 |
| **5** | 2 000/100/1 | 100 | 4 800 | 1,50 |
| **6** | 2 000/100/1 | 100 | 5 000 | 1,51 |
| **7** | 2 000/100/1 | 100 | 5 300 | 1,52 |

Les analyses RMN ¹H (CDCl₃, 500 MHz, 298 K) et ¹³C (CDCl₃, 100 MHz, 298 K) du polymère obtenu pour cet essai ont confirmé la structure du polymère attendu tel que représenté sur le schéma (12).

### ii) Exemples 8 à 9 : Polymérisation d'un mélange de cyclooléfines de formules (A) et (B)

Les cyclooléfines de formule (A) et (B) utilisées dans les exemples 8 et 9 sont respectivement les suivantes :

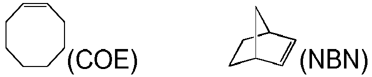

Le cyclooctène (COE) de pureté supérieure à 95 % et le norbornène (NBN) de pureté supérieure à 99 % sont disponibles commercialement auprès de la société Sigma Aldrich. Ces produits ont été au préalable distillés sur CaH₂, avant d'être utilisés dans les exemples 8 et 9.

### Exemple 8: Synthèse d'un polymère comprenant deux groupements terminaux exo-vinylène cyclocarbonate à partir de cyclooctène (COE), de norbornène (NBN) et de bis[(5,5-diméthyl-2-oxo-1,3-dioxolan-4-ylidène)propyl] fumarate (CTA³)

La réaction a été mise en oeuvre selon le schéma (13) suivant, dans un rapport molaire m/n égal à 50/50 et selon le mode opératoire décrit ci-après :

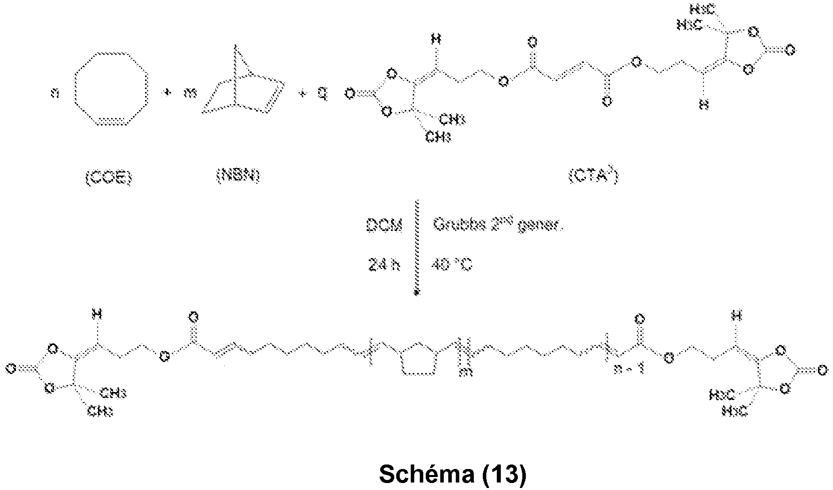

### Mode opératoire :

Dans un ballon de 1000 mL, on a mélangé les cyclooléfines de formules (A) et (B), correspondant à COE (*54,00 mmol*) et NBN (*54,00 mmol*) respectivement, de l'hydroquinone (*0,54 mmole)* et du CH₂Cl₂ sec (50 mL). Le ballon et son contenu ont ensuite été mis sous argon. Le CTA (*5,40 mmol*) de type (C2) a alors été introduit dans le ballon par seringue. Le ballon a alors été immergé dans un bain d'huile à 40 °C puis on a procédé immédiatement à l'ajout, par une canule, du catalyseur G2 (*54 µmol*) en solution dans du CH₂Cl₂ (20 mL). 24 heures après l'addition du catalyseur, le produit a été extrait du ballon après évaporation du solvant sous vide. Le produit a alors été récupéré après précipitation dans le méthanol, filtration et séchage à 20 °C sous vide.

### Résultats :

Le polymère obtenu est liquide à température ambiante.

Les taux de conversion des cyclooléfines de formule (A) et (B) déterminés par RMN (exprimé en %), la masse molaire moyenne en nombre du polymère obtenu (exprimé en gramme par mol) ainsi que la polymolécularité (PDI) du polymère déterminés par SEC. Les résultats ont été consignés dans le tableau 4 suivant :

**Tableau 4 :**

| **Essai n°** | **[A]/[B]/[CTA¹]/[Ru] (mol/mol)** | **Conversion (%)** | **Mn_{SEC} (g/mol)** | **PDI** |
|---|---|---|---|---|
| **8** | 1 000/1 000/100/1 | 100 | 5 000 | 1,60 |

Les analyses RMN ¹H (CDCl₃, 500 MHz, 298 K) et ¹³C (CDCl₃, 100 MHz, 298 K) du polymère obtenu ont confirmé la structure du polymère attendu tel que représenté sur le schéma (13).

### Exemple 9: Synthèse d'un polymère comprenant deux groupements terminaux exo-vinylène cyclocarbonate à partir de cyclooctène (COE), de norbornène (NBN) et de bis[(5,5-diméthyl-2-oxo-1,3-dioxolan-4-ylidène)propyl] fumaramide (CTA⁴)

La réaction a été mise en oeuvre selon le schéma (14) suivant, dans un rapport molaire m/n égal à 50/50 et selon le même mode opératoire que l'exemple 8 :

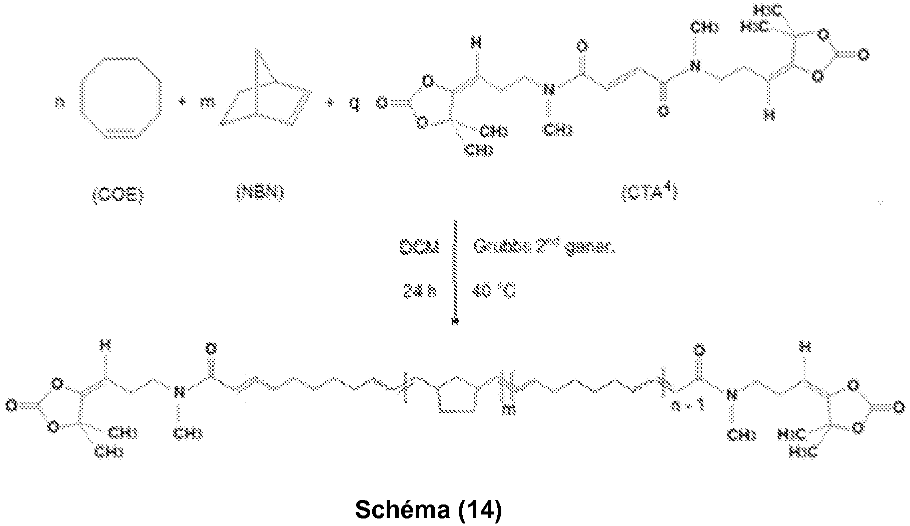

### Résultats :

Le polymère obtenu est liquide à température ambiante.

Les taux de conversion des cyclooléfines de formule (A) et (B) déterminés par RMN (exprimé en %), la masse molaire moyenne en nombre du polymère obtenu (exprimé en gramme par mol) ainsi que la polymolécularité (PDI) du polymère déterminés par SEC. Les résultats ont été consignés dans le tableau 5 suivant :

**Tableau 5 :**

| **Essai n°** | **[A]/[B]/[CTA³]/[Ru] (mol/mol)** | **Conversion (%)** | **Mn_{SEC} (g/mol)** | **PDI** |
|---|---|---|---|---|
| **9** | 1 000/1 000/100/1 | 100 | 5 300 | 1,58 |

Les analyses RMN ¹H (CDCl₃, 500 MHz, 298 K) et ¹³C (CDCl₃, 100 MHz, 298 K) du polymère obtenu ont confirmé la structure du polymère attendu tel que représenté sur le schéma (14).

### Il - Exemple comparatif 10 : Synthèse d'un polymère comprenant deux groupements terminaux méthylène cyclocarbonate à partir de cyclooctène (COE) et de bis[(2-oxo-1,3-dioxolan-4-yl)méthyl] fumarate (CTA⁵)

La réaction a été mise en oeuvre selon le schéma (15) suivant, dans un rapport molaire m/n égal à 0/100 et selon le même mode opératoire que l'exemple 3 :

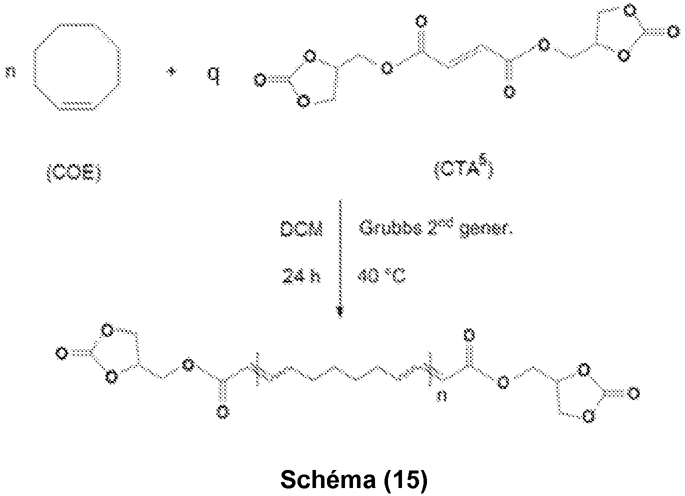

### Résultats :

Le polymère obtenu est solide à température ambiante.

Les résultats du taux de conversion de la cyclooléfine de formule (A) déterminé par RMN (exprimé en %), de la masse molaire moyenne en nombre du polymère obtenu (exprimé en gramme par mol) ainsi que de la polymolécularité (PDI) dudit polymère, déterminés par SEC, ont été consignés dans le tableau 6 suivant :

**Tableau 6 :**

| **Essai n°** | **[A]/[B]/[CTA¹]/[Ru](mol/mol)** | **Conversion (%)** | **Mn_{SEC} (g/mol)** | **PDI** |
|---|---|---|---|---|
| **8** | 1 000/1000/100/1 | 100 | 5 000 | 1,60 |

Les analyses RMN ¹H (CDCl₃, 500 MHz, 298 K) et ¹³C (CDCl₃, 125 MHz, 298 K) du polymère obtenu pour cet essai ont confirmé la structure du polymère attendu tel que représenté sur le schéma (15).

### III - Exemple 11 à 13: Synthèses de polyuréthanes à partir des polyoléfines insaturées des exemples 10, 3 et 8 respectivement :

### Exemple comparatif 11 : Synthèse d'un polyuréthane à partir de la polyoléfine insaturée solide de l'exemple comparatif 10

Il a été mis en réaction à 80°C, séparément et dans un rapport stoechiométrique, la polyoléfine de l'exemple comparatif 10 avec une diamine primaire de type polyéther diamine (JEFFAMINE EDR 148, Huntsman) et ce, jusqu'à disparition complète de la bande infrarouge caractéristique des groupements 1,3-dioxolan-2-one (à 1800 cm⁻¹) et apparition des bandes caractéristiques de la liaison carbamate (bande à 1700 cm⁻¹).

La durée de la réaction relevée lors de la disparation complète de la bande infrarouge caractéristique des groupements 1,3-dioxolan-2-one, était d'environ 12 heures à 80°C.

### Exemple 12 : Synthèse d'un polyuréthane à partir de la polyoléfine insaturée solide de l'exemple 3 selon l'invention

L'exemple 11 a été reproduit en remplaçant la polyoléfine de l'exemple 10 par la polyoléfine de l'exemple 3.

La durée de la réaction relevée lors de la disparation complète de la bande infrarouge caractéristique des groupements 1,3-dioxolan-2-one, était inférieure à 3 heures à 80°C.

### Exemple 13 : Synthèse d'un polyuréthane à partir de la polyoléfine insaturée liquide de l'exemple 8 selon l'invention

L'exemple 11 a été reproduit en remplaçant la polyoléfine de l'exemple 3 par la polyoléfine de l'exemple 8 et en réalisant la réaction à température ambiante (23°C). La durée de la réaction relevée lors de la disparation complète de la bande infrarouge caractéristique des groupements 1,3-dioxolan-2-one, était inférieure à 3 heures à 23°C.

Dans chaque cas, les produits des exemples 12 et 13 ont pû être formulés sous la forme d'un mélange bi-composant présentant des propriétés adhésives satisfaisantes.

## Revendications

1. Polymère hydrocarboné comprenant deux groupements terminaux exo-vinylène cyclocarbonate, ledit polymère hydrocarboné étant de formule (1) suivante : 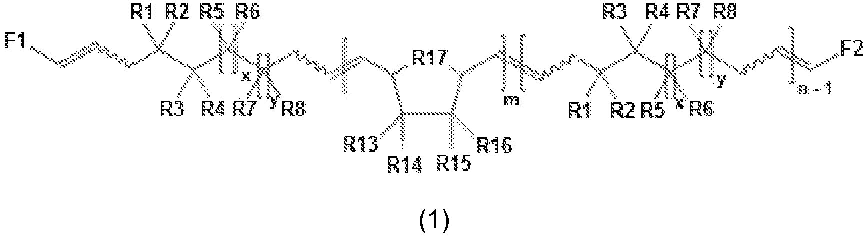 dans laquelle :
• chaque liaison notée 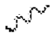 est une liaison simple carbone-carbone orientée géométriquement d'un côté ou de l'autre par rapport à la double liaison (cis ou trans) à laquelle elle est reliée ;
• les groupes R1, R2, R3, R4, R5, R6, R7 et R8, identiques ou différents, sont choisis parmi un atome d'hydrogène, un atome d'halogène, un groupe alkyle, un groupe hétéroalkyle, un groupe alcoxycarbonyle ou un groupe hétéroalcoxycarbonyle,
• au moins un des groupes R1 à R8 pouvant faire partie d'un même cycle hydrocarboné ou hétérocycle, saturé ou insaturé, avec au moins un autre des groupes R1 à R8 ;
• au moins une des paires (R1 ,R2), (R3,R4), (R5,R6) et (R7,R8) pouvant être un groupe oxo ;
• x et y, identiques ou différents, sont des nombres entiers compris dans une fourchette allant de 0 à 5 ;
• les groupes R13, R14, R15 et R16 identiques ou différents, sont choisis parmi un atome d'hydrogène, un atome d'halogène, un groupe alkyle, un groupe hétéroalkyle, un groupe alcoxycarbonyle ou un groupe hétéroalcoxycarbonyle,
• au moins un des groupes R13 à R16 pouvant faire partie d'un même cycle hydrocarboné ou hétérocycle, saturé ou insaturé, avec au moins un autre des groupes R13 à R16 ;
• le groupe R17 est CH₂, O, S, C(=O) ou NR₀, R₀ étant un groupe alkyle comportant de 1 à 22 ; et
• n est un nombre entier supérieur ou égal à 2 et m est un nombre entier supérieur ou égal à 0, le rapport molaire m/n étant compris dans une fourchette allant de 0/100 à 90/10 ; n et m étant en outre tels que la masse molaire moyenne en nombre Mn du polymère hydrocarboné de formule (1) est comprise dans une fourchette allant de 400 à 50 000 g/mol ;
• F1 est représenté par la formule suivante : 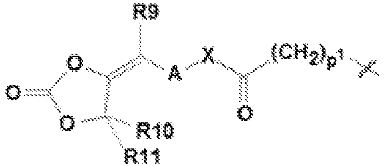
• et F2 est représenté par la formule suivante : 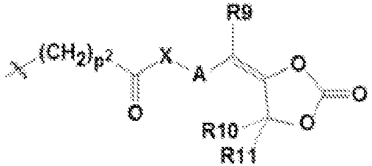 dans lesquelles :
p1 et p2, identiques ou différents, représentent chacun un nombre entier égal à 0, 1, 2 ou 3 ;
X est un atome d'oxygène ou un groupe azoté NR12 où R12 est un groupe alkyle en C1-C6 ;
A est un groupe alkylène en C1-C6 ;
R9 est un atome d'hydrogène, un groupe alkyle en C1-C6, un groupe alkyle en C1-C6 oxyalkyléné par un ou plusieurs groupes oxyalkylène en C1-C6, un groupe cycloalkyle en C5-C6, un groupe phényle ou un groupe alkylphényle avec une chaîne alkyle en C1-C4 ;
R10 et R11, identiques ou différents, représentent chacun un atome d'hydrogène, un groupe alkyle en C1-C6, un groupe alkyle en C1-C6 oxyalkyléné par un ou plusieurs groupes oxyalkylène en C1-C6, un groupe cycloalkyle en C5-C6, un groupe phényle ou un groupe alkylphényle avec une chaîne alkyle en C1-C4.

2. Polymère hydrocarboné comprenant deux groupements terminaux exo-vinylène cyclocarbonate selon la revendication 1, dans lequel m est égal à 0, le polymère étant de formule (2) suivante : 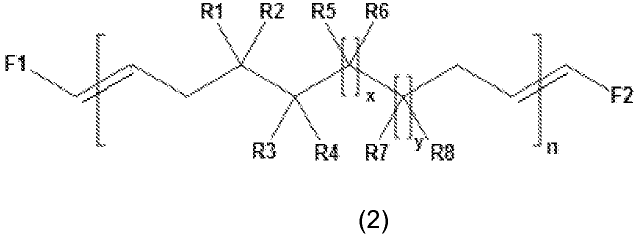 dans laquelle : x, y, n, F1, F2, R1, R2, R3, R4, R5, R6, R7 et R8 étant tels que définis dans la revendication 1.

3. Polymère hydrocarboné comprenant deux groupements terminaux exo-vinylène cyclocarbonate selon la revendication 1 ou 2, **caractérisé en ce que** X est un atome d'oxygène.

4. Polymère hydrocarboné comprenant deux groupements terminaux exo-vinylène cyclocarbonate selon la revendication 1 ou 2, **caractérisé en ce que** X est un groupe NR₁₂ dans lequel R₁₂ est tel que défini dans la revendication 1 ou 2

5. Polymère hydrocarboné comprenant deux groupements terminaux exo-vinylène cyclocarbonate selon l'une quelconque des revendications 1, 2 et 4, **caractérisé en ce que** R12 est un groupe méthyle.

6. Polymère hydrocarboné comprenant deux groupements terminaux exo-vinylène cyclocarbonate selon l'une quelconque des revendications 1 à 5, **caractérisé en ce que** R9 est un atome d'hydrogène, R10 et R11 sont des groupes méthyle, et avec p1 = 0 ou p2 = 0.

7. Polymère hydrocarboné comprenant deux groupements terminaux exo-vinylène cyclocarbonate selon l'une quelconque des revendications 1 à 5, **caractérisé en ce que** p1 = p2 = 0.

8. Procédé de préparation d'au moins un polymère hydrocarboné comprenant deux groupements terminaux exo-vinylène cyclocarbonate selon l'une des revendications 1 à 7, ledit procédé comprenant au moins une étape de polymérisation par ouverture de cycle par métathèse, en présence :
- d'au moins un catalyseur de métathèse,
- d'au moins un agent de transfert de chaîne mono ou di-exovinylène cyclocarbonate respectivement de formule (C1) ou (C2) suivante : 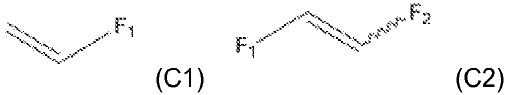 dans lesquelles :
• F1 et F2 sont tels que définis dans l'une quelconque des revendications 1 à 7, et
• la liaison 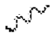 est une liaison simple carbone-carbone orientée géométriquement d'un côté ou de l'autre par rapport à la double liaison (cis ou trans) dans la formule (C2) ;
- d'au moins un composé de formule (A) suivante : 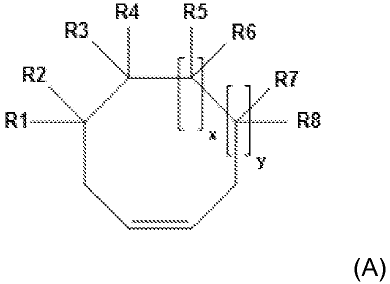 dans laquelle :
• les groupes R1, R2, R3, R4, R5, R6, R7 et R8, x et y sont tels que définis dans l'une quelconque des revendications 1 à 7 ; et
- d'éventuellement au moins un composé de formule (B) : 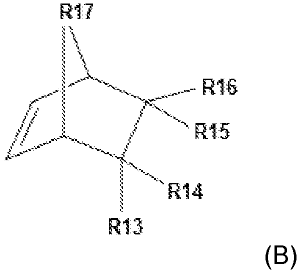 dans laquelle :
• les groupes R13, R14, R15 et R16 sont tels que définis dans l'une quelconques des revendications 1 à 7 ;
pendant une durée de réaction allant de 2 à 24 heures et à une température comprise dans une fourchette allant de 20 à 60°C.

9. Procédé de préparation selon la revendication 8, ledit procédé étant tel que le rapport molaire du CTA de formule (C1) sur le composé de formule (A), ou sur la somme des composés de formules (A) et (B) si le composé de formule (B) est présent, est compris dans une fourchette allant de 1.10⁻³ à 1,0 ou le rapport molaire du CTA de formule (C2) sur le composé de formule (A), ou sur la somme des composés de formules (A) et (B) si le composé de formule (B) est présent, est compris dans une fourchette allant de 0,5.10⁻³ à 0,5.

10. Procédé de préparation de polyuréthane comprenant la réaction la réaction d'au moins un polymère hydrocarboné de formule (1) selon l'une quelconque des revendications 1 à 7, avec au moins un composé comprenant au moins un groupement amine.

11. Polyuréthanes susceptibles d'être obtenus par le procédé de préparation selon la revendication 10.

12. Composé de formule (C2) tel que défini dans la revendication 8.

## Patentansprüche

1. Kohlenwasserstoff-Polymer, umfassend zwei Exovinylen-Cyclocarbonat-Endgruppen, wobei das Kohlenwasserstoff-Polymer folgende Formel (1) aufweist: 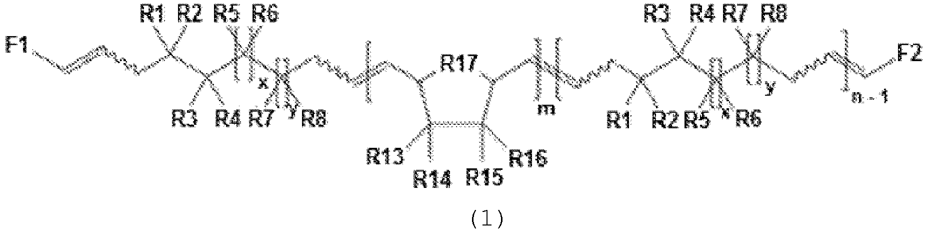 worin:
• jede 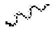 eingezeichnete Bindung eine Kohlenstoff-Kohlenstoff-Einfachbindung ist, die auf der einen oder der anderen Seite zur (Cis- oder Trans-)Doppelbindung, mit der sie verbunden ist, geometrisch ausgerichtet ist;
• die Gruppen R1, R2, R3, R4, R5, R6, R7 und R8 gleich oder verschieden sein können und unter einem Wasserstoffatom, einem Halogenatom, einer Alkylgruppe, einer Heteroalkylgruppe, einer Alkoxycarbonylgruppe oder einer Heteroalkoxycarbonylgruppe ausgewählt werden,
• mindestens eine der Gruppen R1 bis R8 zu demselben, gesättigten oder ungesättigten, Kohlenwasserstoff- oder Heterozyklus mit mindestens einer anderen der Gruppen R1 bis R8 gehören kann;
• mindestens eines der Paare (R1,R2), (R3,R4), (R5,R6) und (R7,R8) eine Oxogruppe sein kann;
• x und y gleich oder verschieden sein können und Ganzzahlen in einem Bereich zwischen 0 und 5 sind;
• die Gruppen R13, R14, R15 und R16 gleich oder verschieden sein können und unter einem Wasserstoffatom, einem Halogenatom, einer Alkylgruppe, einer Heteroalkylgruppe, einer Alkoxycarbonylgruppe oder einer Heteroalkoxycarbonylgruppe ausgewählt werden;
• mindestens eine der Gruppen R13 bis R16 zu demselben, gesättigten oder ungesättigten, Kohlenwasserstoff- oder Heterozyklus mit mindestens einer anderen der Gruppen R13 bis R16 gehören kann;
• die Gruppe R17 CH₂, 0, S, C (=O) oder NR₀ ist, wobei R₀ eine Alkylgruppe mit 1 bis 22 Kohlenstoffatomen ist; und
• n eine Ganzzahl ist, die größer oder gleich der Zahl 2 ist, und m eine Ganzzahl ist, die größer oder gleich der Zahl 0 ist, wobei das Molverhältnis m/n in einem Bereich von 0/100 bis 90/10 liegt; n und m sind dabei so groß, dass die mittlere molare Masse Mn des Kohlenwasserstoff-Polymers der Formel (1) in einem Bereich von 400 bis 50.000 g/mol liegt;
• F1 durch die folgende Formel dargestellt wird: 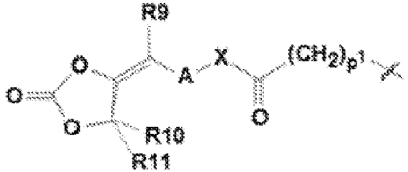
• und F2 durch die folgende Formel dargestellt wird: 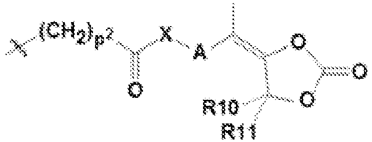 worin:
p1 und p2 gleich oder verschieden sein können und jeweils eine ganze Zahl gleich 0, 1, 2 oder 3 darstellen;
X ein Sauerstoffatom oder eine Stickstoffgruppe NR12 ist, wobei R12 eine C1-C6-Alkylgruppe ist;
A eine C1-C6-Alkylengruppe ist;
R9 ein Wasserstoffatom, eine C1-C6-Alkylgruppe, eine C1-C6-Alkylgruppe, die durch eine oder mehrere C1-C6-Oxyalkylengruppen oxyalkyliert ist, eine C5-C6-Zykloalkylgruppe, eine Phenylgruppe oder eine Alkylphenylgruppe mit einer C1-C4-Alkylkette ist;
R10 und R11 gleich oder verschieden sein können und jeweils ein Wasserstoffatom, eine C1-C6-Alkylgruppe, eine C1-C6-Alkylgruppe, die durch eine oder mehrere C1-C6-Oxyalkylengruppen oxyalkyliert ist, eine C5-C6-Zykloalkylgruppe, eine Phenylgruppe oder eine Alkylphenylgruppe mit einer C1-C4-Alkylkette darstellen.

2. Kohlenwasserstoff-Polymer, umfassend zwei Exovinylen-Cyclocarbonat-Endgruppen nach Anspruch 1, bei dem m gleich 0 ist, wobei das Polymer folgende Formel (2) aufweist: 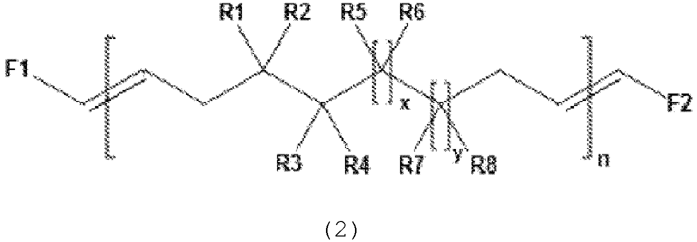 bei der: x, y, n, F1, F2, R1, R2, R3, R4, R5, R6, R7 und R8 wie in Anspruch 1 definiert sind.

3. Kohlenwasserstoff-Polymer, umfassend zwei Exovinylen-Cyclocarbonat-Endgruppen nach Anspruch 1 oder 2, **dadurch gekennzeichnet, dass** X ein Sauerstoffatom ist.

4. Kohlenwasserstoff-Polymer, umfassend zwei Exovinylen-Cyclocarbonat-Endgruppen nach Anspruch 1 oder 2, **dadurch gekennzeichnet, dass** X eine Gruppe NR12 ist, wobei R12 wie in Anspruch 1 oder 2 definiert ist.

5. Kohlenwasserstoff-Polymer, umfassend zwei Exovinylen-Cyclocarbonat-Endgruppen nach einem beliebigen der vorstehenden Ansprüche 1, 2 und 4, **dadurch gekennzeichnet, dass** R12 eine Methylgruppe ist.

6. Kohlenwasserstoff-Polymer, umfassend zwei Exovinylen-Cyclocarbonat-Endgruppen nach einem der vorstehenden Ansprüche 1 bis 5, **dadurch gekennzeichnet, dass** R9 ein Wasserstoffatom ist, R10 und R11 Methylgruppen sind, wobei p1 = 0 oder p2 = 0.

7. Kohlenwasserstoff-Polymer, umfassend zwei Exovinylen-Cyclocarbonat-Endgruppen nach einem der vorstehenden Ansprüche 1 bis 5, **dadurch gekennzeichnet, dass** p1 = p2 = 0 ist.

8. Verfahren zur Herstellung mindestens eines Kohlenwasserstoff-Polymers, umfassend zwei Exovinylen-Cyclocarbonat-Endgruppen nach einem der vorstehenden Ansprüche 1 bis 7, wobei dieses Verfahren mindestens eine Phase der Polymerisation durch Zyklusöffnung-Metathese aufweist, mit:
- mindestens einem Metathese-Katalysator,
- mindestens einem Mono- oder Di-Exovinylen-Cyclocarbonat-Kettentransfermittel, jeweils nach folgender Formel (C1) oder (C2): 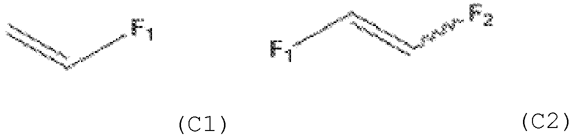 worin:
• F1 und F2 wie in einem beliebigen der vorstehenden Ansprüche 1 bis 7 definiert sind, und
• die Bindung eine Kohlenstoff-Kohlenstoff-Einfachbindung ist, die auf der einen oder der anderen Seite zur (Cis- oder Trans-)Doppelbindung in der Formel (C2) geometrisch ausgerichtet ist;
mindestens eine Verbindung der folgenden Formel (A): 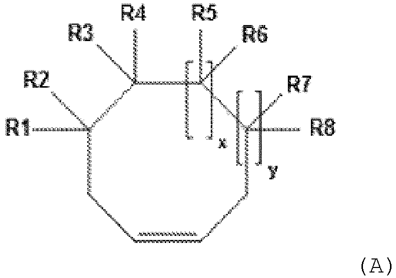
bei der:
• die Gruppen R1, R2, R3, R4, R5, R6, R7 und R8, x und y wie in einem beliebigen der vorstehenden Ansprüche 1 bis 7 definiert sind; und
• eventuell mindestens eine Verbindung der Formel (B): 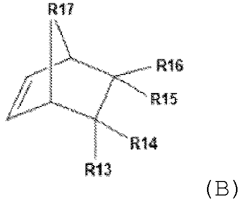
bei der:
• die Gruppen R13, R14, R15 und R16 wie in einem beliebigen der vorstehenden Ansprüche 1 bis 7 definiert sind;
während einer Reaktionszeit von 2 bis 24 Stunden und bei einer Temperatur in einem Bereich von 20 bis 60 °C.

9. Herstellungsverfahren nach Anspruch 8, wobei dieses Verfahren derart ist, dass das Molverhältnis von CTA mit der Formel (C1) zu der Verbindung mit der Formel (A) oder der Summe der Verbindungen mit den Formeln (A) und (B) bei Vorhandensein der Verbindung mit der Formel (B) in einem Bereich zwischen 1.10⁻³ und 1,0 liegt, oder dass das Molverhältnis von CTA mit der Formel (C2) zu der Verbindung mit der Formel (A) oder der Summe der Verbindungen mit den Formeln (A) und (B) bei Vorhandensein der Verbindung mit der Formel (B) in einem Bereich zwischen 0,5·10⁻³ und 0,5 liegt.

10. Verfahren zur Herstellung von Polyurethan, umfassend die Reaktion mindestens eines Kohlenwasserstoff-Polymers der Formel (1) nach einem beliebigen der vorstehenden Ansprüche 1 bis 7, wobei mindestens eine Verbindung mindestens eine Amingruppe enthält.

11. Polyurethane, die durch das Herstellungsverfahren nach Anspruch 10 erhalten werden können.

12. Verbindung der Formel (C2), wie in Anspruch 8 definiert.

## Claims

1. A hydrocarbon polymer comprising two exo-vinylene cyclocarbonate end groups, said hydrocarbon polymer being of formula (1) below: 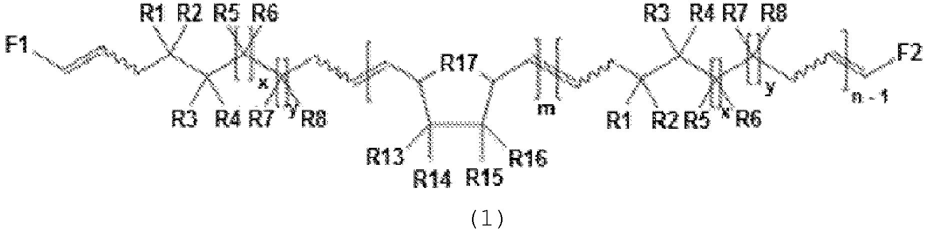 wherein:
• each noted bond 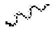 is a single carbon-carbon bond oriented geometrically on one side or the other relative to the double bond (cis or trans) to which it is bonded;
• groups R1, R2, R3, R4, R5, R6, R7 and R8, which may be identical or different, are selected from a hydrogen atom, a halogen atom, an alkyl group, a heteroalkyl group, an alkoxycarbonyl group or a heteroalkoxycarbonyl group;
• at least one of groups R1 to R8 possibly forming part of the same saturated or unsaturated hydrocarbon-based ring or heterocycle, with at least one other from groups R1 to R8;
• at least one of the (R1,R2), (R3,R4), (R5,R6) and (R7,R8) pairs, possibly being an oxo group;
• x and y, which may be identical or different, are integers ranging from 0 to 5;
• groups R13, R14, R15 and R16, which may be identical or different, are selected from a hydrogen atom, a halogen atom, an alkyl group, a heteroalkyl group, an alkoxycarbonyl group or a heteroalkoxycarbonyl group,
• at least one of groups R13 to R16 possibly forming part of the same saturated or unsaturated hydrocarbon-based ring or heterocycle, with at least one other from groups R13 to R16;
• group R17 is composed of CH₂, O, S, C(=O) or NR₀, R₀ being an alkyl group comprising from 1 to 22; and
• n is an integer greater than or equal to 2 and m is an integer greater than or equal to 0, the molar ratio m/n ranging from 0/100 to 90/10, n and m further being such that the number-average molar mass Mn of the hydrocarbon polymer of formula (1) ranges from 400 to 50000 g/mol;
• F1 is represented by the following formula: 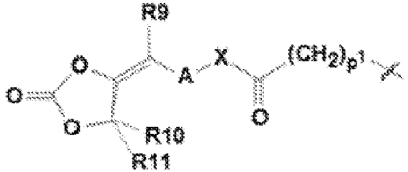
• and F2 is represented by the following formula: 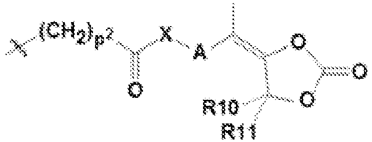 wherein:
p1 and p2, which may be identical or different, each represent an integer equal to 0, 1, 2 or 3;
X is an oxygen atom or a nitrogen group NR12 where R12 is a C1-C6 alkyl group;
A is a C1-C6 alkylene group;
R9 is a hydrogen atom, a C1-C6 alkyl group, a C1-C6 alkyl group oxyalkylenated with one or more C1-C6 oxyalkylene groups, a C5-C6 cycloalkyl group, a phenyl group or an alkylphenyl group with a C1-C4 alkyl chain;
R10 and R11, which may be identical or different, each represent a hydrogen atom, a C1-C6 alkyl group, a C1-C6 alkyl group oxyalkylenated with one or more C1-C6 oxyalkylene groups or a C5-C6 cycloalkyl group, a phenyl group or an alkylphenyl group with a C1-C4 alkyl chain.

2. A hydrocarbon polymer comprising two exo-vinylene cyclocarbonate end groups according to claim 1, wherein m is 0, the polymer being of formula (2) below: 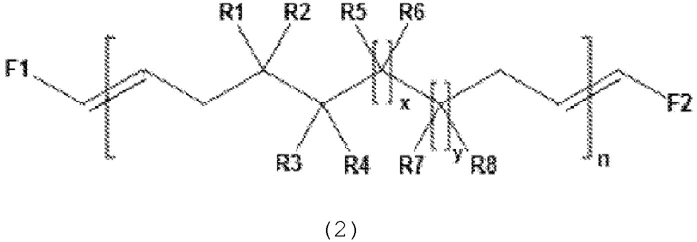 wherein: x, y, n, F1, F2, R1, R2, R3, R4, R5, R6, R7 and R8 are as defined in claim 1.

3. Hydrocarbon polymer comprising two exo-vinylene cyclocarbonate end groups according to claim 1 or 2, **characterized in that** X is an oxygen atom.

4. Hydrocarbon polymer comprising two exo-vinylene cyclocarbonate end groups according to claim 1 or 2, **characterized in that** X is an NR12 group wherein R12 is as defined in claim 1 or 2

5. Hydrocarbon polymer comprising two exo-vinylene cyclocarbonate end groups according to any one of claims 1, 2 and 4, **characterized in that** R12 is a methyl group.

6. Hydrocarbon polymer comprising two exo-vinylene cyclocarbonate end groups according to any one of Claims 1 to 5, **characterized in that** R9 is a hydrogen atom, R10 and R11 are methyl groups, and p1 = 0 or p2 = 0.

7. Hydrocarbon polymer comprising two exo-vinylene cyclocarbonate end groups according to any one of Claims 1 to 5, **characterized in that** p1 = p2 = 0.

8. Process for the preparation of at least one hydrocarbon polymer comprising two endo-vinylene cyclocarbonate end groups according to one of claims 1 to 7, said process comprising at least one metathesis ring opening polymerization step, in the presence of:
- of at least one metathesis catalyst,
- at least one mono or di-exovinylene cyclocarbonate chain transfer agent of formula (C1) or (C2) below: 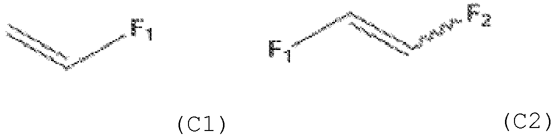 wherein:
• F1 and F2 are as defined in any one of claims 1 to 7, and
• the bond is a carbon-carbon single bond geometrically oriented on one side or the other relative to the double bond (cis or trans) in formula (C2);
- of at least one compound of formula (A) below: 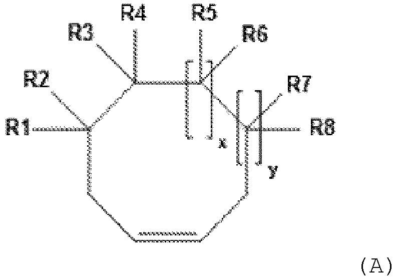 wherein:
• groups R1, R2, R3, R4, R5, R6, R7 and R8, x and y are as defined in any one of claims 1 to 7; and
• of optionally at least one compound of formula (B): 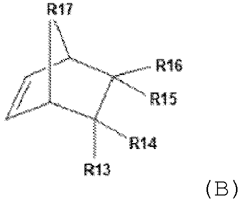
wherein:
• groups R13, R14, R15 and R16, x and y are as defined in any one of claims 1 to 7;
during a reaction time ranging from 2 to 24 hours and at a temperature ranging from 20 to 60 °C.

9. Preparation process according to claim 8, said process being such that the molar ratio of the CTA of formula (C1) to the compound of formula (A), or the sum of the compounds of formulas (A) and (B) if the compound of formula (B) is present, is within a range of 1.10⁻³ to 1.0 or the molar ratio of the CTA of formula (C2) to the compound of formula (A), or to the sum of the compounds of formulas (A) and (B) if the compound of formula (B) is present, is within a range of 0.5.10⁻³ to 0.5.

10. A process for preparing polyurethane comprising the reaction of at least one hydrocarbon polymer of formula (1) according to any one of claims 1 to 7, with at least one compound comprising at least one amine group.

11. Polyurethanes obtainable by the preparation process according to claim 10.

12. A compound of formula (C2) as defined in claim 8.
